(19) Europäisches Patentamt
European Patent Office
Office européen des brevets

(11) EP 3 121 160 B1

(12) **EUROPEAN PATENT SPECIFICATION**

(45) Date of publication and mention of the grant of the patent:
**01.01.2020 Bulletin 2020/01**

(21) Application number: **15765516.8**

(22) Date of filing: **19.03.2015**

(51) Int Cl.:
***C07C 41/01*** *(2006.01)* ***C07C 41/16*** *(2006.01)*
***C07C 43/225*** *(2006.01)* ***C07C 327/26*** *(2006.01)*

(86) International application number:
**PCT/JP2015/058381**

(87) International publication number:
**WO 2015/141811 (24.09.2015 Gazette 2015/38)**

(54) **METHOD FOR PRODUCING COMPOUND HAVING OXYDIFLUOROMETHYLENE SKELETON**

VERFAHREN ZUR HERSTELLUNG EINER VERBINDUNG MIT OXYDIFLUORMETHYLENGERÜST

PROCÉDÉ DE PRODUCTION DE COMPOSÉ AYANT UN SQUELETTE OXYDIFLUOROMÉTHYLÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.03.2014 JP 2014058909**

(43) Date of publication of application:
**25.01.2017 Bulletin 2017/04**

(73) Proprietor: **Daikin Industries, Ltd.**
**Osaka 530-8323 (JP)**

(72) Inventors:
- **YAMAMOTO, Yuuki**
  **Osaka 530-8323 (JP)**
- **NOSE, Masatoshi**
  **Osaka 530-8323 (JP)**
- **NAMIKAWA, Takashi**
  **Osaka 530-8323 (JP)**
- **NOMURA, Takashi**
  **Osaka 530-8323 (JP)**
- **ISHIHARA, Sumi**
  **Osaka 530-8323 (JP)**
- **YOSHIYAMA, Asako**
  **Osaka 530-8323 (JP)**
- **KISHIMOTO, Masayuki**
  **Osaka 530-8323 (JP)**
- **SUYAMA, Makoto**
  **Osaka 530-8323 (JP)**
- **ADACHI, Kenji**
  **Osaka 530-8323 (JP)**
- **KISHIKAWA, Yosuke**
  **Osaka 530-8323 (JP)**

(74) Representative: **Hoffmann Eitle**
**Patent- und Rechtsanwälte PartmbB**
**Arabellastraße 30**
**81925 München (DE)**

(56) References cited:
**WO-A1-01/96263**
**JP-A- H1 017 544**
**JP-A- H10 204 016**
**JP-A- 2013 241 401**
**US-A1- 2011 233 466**

- **S. ROZEN, ET AL.: "Conversion of esters to [alpha],[alpha]-difluoro ethers using bromine trifluoride", JOURNAL OF THE CHEMICAL SOCIETY, CHEMICAL COMMUNICATIONS, no. 23, 1 January 1993 (1993-01-01), pages 1761-1762, XP055418348, Royal Society of Chemistry, Cambridge, GB ISSN: 0022-4936, DOI: 10.1039/c39930001761**
- **T. NAKAMURA, ET AL.: "Mercaptomethylation of aromatics", SYNTHETIC COMMUNICATIONS, vol. 29, no. 2, January 1999 (1999-01), pages 201-210, XP055418619, Taylor & Francis, London, GB ISSN: 0039-7911, DOI: 10.1080/00397919908085758**
- **HARTKE, K. ET AL.: 'ALPHA, BETA-ACETYLENIC DITHIO AND THIONO ESTERS' TETRAHEDRON LETTERS vol. 30, no. 9, 1989, pages 1073 - 1076, XP055084574**
- **THE CHEMICAL SOCIETY OF JAPAN JIKKEN KAGAKU KOZA 13 YUKI KAGOBUTSU NO GOSEI I -TANKA SUISO HALOGEN KABUTSU 2004, pages 104 - 106, XP008185016**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 121 160 B1

## Description

### Technical Field

The present invention relates to a method for producing a compound having an oxydifluoromethylene skeleton.

### Background Art

Compounds having an oxydifluoromethylene skeleton are useful as, for example, a liquid crystal material, a medicinal drug, and an intermediate thereof, and studies have been made on various production methods.

For example, Patent Literature (PTL) 1 below discloses a method for producing a compound having an oxydifluoromethylene skeleton with a halogen atom at a terminal end. This compound is a useful substance as an intermediate of, for example, a liquid crystal material. The method comprises the following steps.

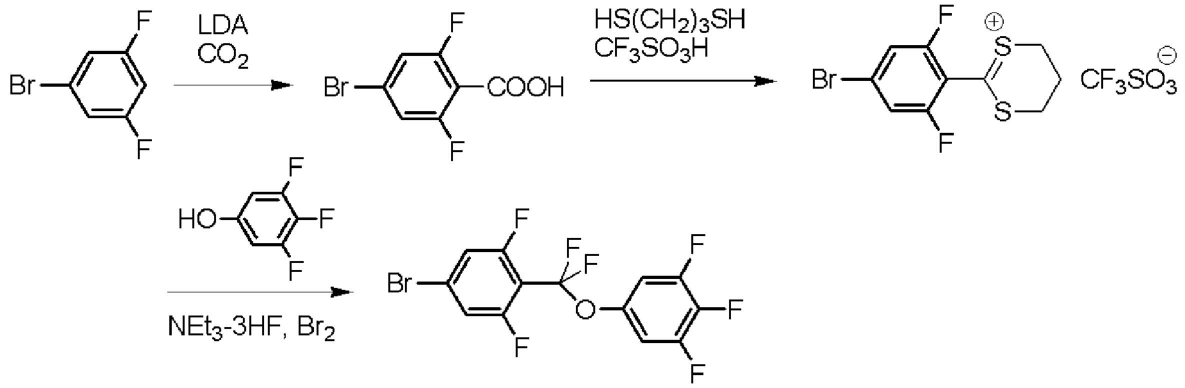


However, the production process of this method requires three steps and is thus complicated. Moreover, this method has problems in that the intermediate products have unsatisfactory stability, and the handling is difficult.

Patent Literature (PTL) 2 below discloses a method for producing a compound having an oxydifluoromethylene skeleton with a lower alkyl group at a terminal end. This method comprises the following steps.

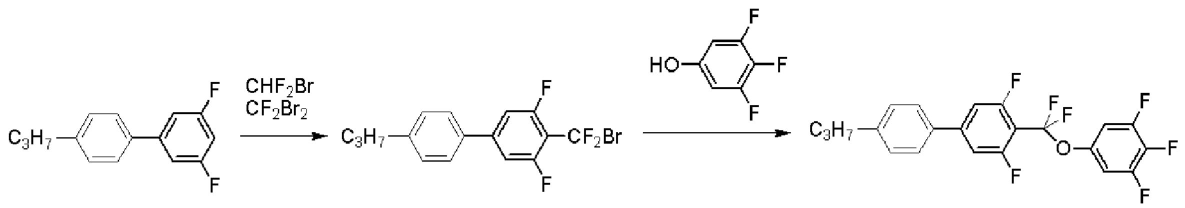


As reactants, the method uses expensive, highly toxic dibromodifluoromethane, as well as bromodifluoromethane, which is a gas component that has a high ozone destruction coefficient; thus, handling at the time of reaction is difficult, and in addition, the yield is low, and the removal of a large amount of by-products is necessary.

Patent Literature (PTL) 3 below discloses a method for producing a compound having an oxydifluoromethylene skeleton with a halogen atom at a terminal end. This method comprises the following steps.

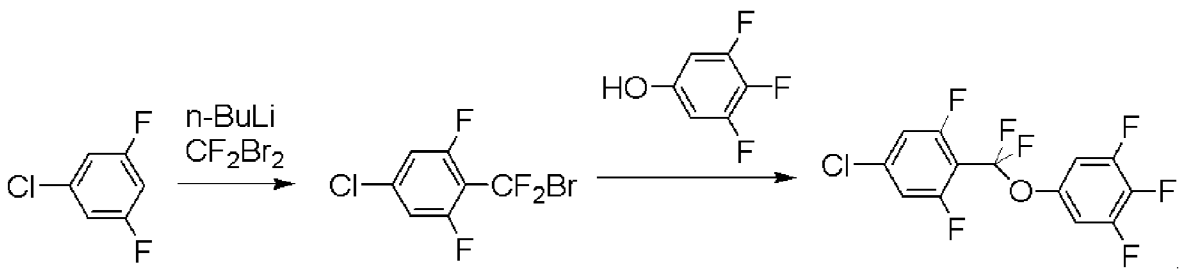


However, this method also has problems in that it uses expensive, highly toxic dibromodifluoromethane, and

in addition, the yield is low, and the removal of a large amount of by-products is necessary; thus, further improvements are required to industrially practice the method.

Patent Literature (PTL) 4 describes liquid-crystalline compounds having at least three fluorine-substituted benzene rings, a terminal trifluoromethyl group and at least one $-CF_2O$-bridge, which are synthesized using boronic acid derivatives as intermediates.

Rozen et. al. describe the conversion of Esters to $\alpha$, $\alpha$-difluoro ethers using bromine trifluoride in J.Chem.Soc., Chem. Commun., 1993. Nakamura et. al. describe the mercaptomethylation of aromatics in Synthetic Communications, 29(2), 201-210 (1999). The mercaptomethylation is effected by metalation of aromatics with magnesium or butyllithium/magnesium bromide diethyl etherate, followed by addition of O-propyl chlorothioformiate and reduction with $LiALH_4$ or $NaBH_4$.

Citation List

Patent Literature

PTL 1: JP2003-525286A
PTL 2: JP2003-261478A
PTL 3: JP2013-241401A
PTL 4: US 2011/0233466 A1

Summary of Invention

Technical Problem

The present invention was made in view of the foregoing state of the art, and a primary object of the present invention is to provide a method for producing a compound having an oxydifluoromethylene skeleton at a high yield by a simple process without using difficult-to-handle starting materials, such as dibromodifluoromethane, and without generating difficult-to-separate impurities.

Solution to Problem

The present inventors conducted extensive research to achieve the above object, and in the course of the research, the inventors found that a method comprising reacting a specific compound called a "thionocarboxylic acid ester" as a starting material with a compound represented by Formula: $IF_n$ (wherein n represents a natural number of 1 to 5) (in this specification, this compound is sometimes simply referred to as "fluorinating agent $IF_n$") (e.g., iodine pentafluoride) is capable of producing a high-purity compound having an oxydifluoromethylene skeleton at a high yield by a simple purification step while reducing the generation of difficult-to-separate impurities.

The present inventors further found regarding the thionocarboxylic acid ester used as a starting material in the above method that the use of a method comprising reacting a halogenated thiocarbonyl compound as a starting material with a specific compound makes it possible to produce the thionocarboxylic acid ester at a high yield with the use of relatively safe starting materials under mild reaction conditions. The inventors further found that a combination of this reaction with a fluorination reaction performed with the fluorinating agent $IF_n$ (e.g., iodine pentafluoride) makes it possible to produce a compound having an oxydifluoromethylene skeleton at a high yield by a relatively simple reaction step using highly safe starting materials without generating difficult-to-separate impurities.

The present inventors further found that the compound obtained by this method is useful by itself as a liquid crystal material or the like. Additionally, the present inventors found that a further reaction between the compound obtained by this method, which contains a reactive atom or group, and a specific aromatic boronic acid compound allows for the introduction of various groups, such as an aromatic ring group, in high yield, making the conversion possible into, for example, various compounds that are useful as a medicinal drug, a liquid crystal material, and the like.

Based on the above findings, the present inventors conducted further research. The present invention has thus been accomplished.

Specifically, the present invention provides the methods for producing a compound having an oxydifluoromethylene skeleton as defined in the claims.

The following describes in detail the method for producing a compound having an oxydifluoromethylene skeleton of the present invention.

(I) Fluorination Reaction Step of Thionocarboxylic Acid Ester

In the present invention, a thionocarboxylic acid ester represented by Formula (1) is used as a starting material:

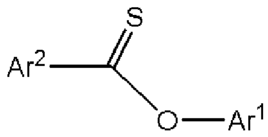

(1)

wherein $Ar^1$ is an aromatic ring group, cycloalkyl or alkyl, each optionally substituted, and $Ar^2$ is (a) an optionally substituted aromatic ring group, (b) an optionally substituted cycloalkyl, or (c) an alkyl optionally substituted by one or more groups selected from cycloalkyl, fluoroalkyl, alkoxy, fluoroalkoxy, halogen, -SF5, and a boronic acid group, and reacted with a fluorinating agent $IF_n$ to give a compound having an oxydifluoromethylene skeleton represented by Formula (2) :

$$Ar^2\text{-}CF_2O\text{-}Ar^1 \qquad (2),$$

wherein $Ar^1$ and $Ar^2$ are as defined above.

The fluorinating agent $IF_n$ is particularly preferably $IF_5$.

In this method, a specific compound, i.e., a thionocarboxylic acid ester represented by Formula (1) above, is used as a starting material and subjected to fluorination with a specific fluorinating agent, i.e., the fluorinating agent $IF_n$ (particularly preferably iodine pentafluoride ($IF_5$)). In this manner, a high-purity compound having an oxydifluoromethylene skeleton is obtained at a high yield while reducing the generation of difficult-to-separate impurities. Moreover, iodine pentafluoride used as a fluorinating agent is a non-explosive, easy-to-handle liquid having a boiling point of 100.5°C and a melting point of 9.4°C, and is an industrially useful fluorinating agent.

Among the groups represented by $Ar^1$ and $Ar^2$ in Formula (1) above, examples of optionally substituted aromatic ring groups include optionally substituted phenyl, optionally substituted naphthyl, optionally substituted pyridyl, and the like. Examples of cycloalkyl in an optionally substituted cycloalkyl group include cycloalkyl groups having 4 to 6 carbon atoms, such as cyclohexyl, cyclopentyl, and cyclobutyl. Examples of alkyl in an optionally substituted alkyl group include straight or branched alkyl groups having 1 to 6 carbon atoms, such as methyl, ethyl, n-propyl, isopropyl, n-butyl, isobutyl, tert-butyl, sec-butyl, n-pentyl, isopentyl, neopentyl, n-hexyl, isohexyl, and 3-methylpentyl. Except for the optional substituents in the optionally substituted alkyl group represented by $Ar^2$, which can be selected from cycloalkyl, fluoroalkyl, alkoxy, fluoroalkoxy, halogen, -SF5, and a boronic acid group, the substituents on these groups are not particularly limited as long as they are inactive to a fluorination reaction performed with the fluorinating agent $IF_n$ mentioned below (particularly preferably iodine pentafluoride), and also as long as they are inactive to the reaction described below between a halogenated thiocarbonyl compound represented by Formula (3) and a compound represented by Formula (4), when the thionocarboxylic acid ester represented by Formula (1) is produced through this reaction. Specific examples of such substituents include alkyl groups, cycloalkyl groups, fluoroalkyl groups, alkoxy groups, fluoroalkoxy groups, aromatic ring groups, halogen atoms, $-SF_5$, boronic acid groups, and the like. In this specification, the number of substituents may be one or more. In this specification, "lower" indicates that the carbon number is six or less.

Of these, examples of alkyl and cycloalkyl groups include the same groups mentioned above. Examples of fluoroalkyl groups include fluoroalkyl groups in which some or all of the hydrogen atoms in straight or branched lower alkyl having 1 to 6 carbon atoms are replaced with fluorine. Examples of alkoxy groups include straight or branched lower alkoxy groups having 1 to 6 carbon atoms. Examples of fluoroalkoxy groups include fluoroalkoxy groups in which some or all of the hydrogen atoms in straight or branched lower alkyl having 1 to 6 carbon atoms are replaced with fluorine. Examples of aromatic ring groups as substituents include phenyl, naphthyl, and the like; these aromatic ring groups may further contain a substituent that is inactive to the reaction.

A boronic acid group is represented by Formula (a).

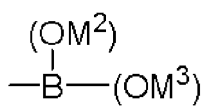

(a)

In this boronic acid group, $M^2$ and $M^3$ are identical or different, and each represents a hydrogen atom, a lower alkyl group, or a monovalent metal atom. Further, $M^2$ and $M^3$ may bond to each other to form a divalent aliphatic

hydrocarbon group, and taken together with the boron atom, may form a ring structure. Examples of such a divalent aliphatic hydrocarbon group include straight or branched aliphatic hydrocarbon groups having 2 to 6 carbon atoms.

Among the atoms or groups represented by $M^2$ and $M^3$ in Formula (a) above representing a boronic acid group, examples of a lower alkyl group include the same alkyl groups mentioned above as substituents. Examples of a monovalent metal atom include alkali metals, such as Na, K, and Li.

Specific examples of boronic acid groups represented by Formula (a) in which $M^2$ and $M^3$ bond to each other to form a divalent aliphatic hydrocarbon group include groups represented by the following formulas.

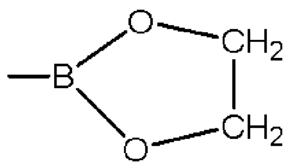

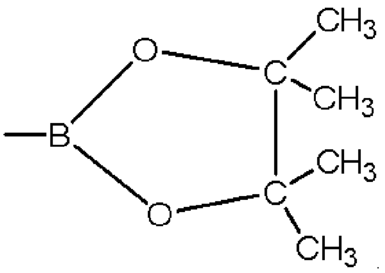

Each group shown above may be easily obtained through a reaction between, for example, a boronic acid and ethylene glycol or pinacol.

When a compound substituted with a boronic acid group is used as a starting material, the compound may be sometimes partly dehydration-condensed. The present invention may also use a compound in such a condensed state, such as a trimer, as a starting material.

In the present invention, $Ar^1$ and $Ar^2$ each preferably represent an optionally substituted aromatic ring group.

Of the groups mentioned above represented by $Ar^1$, specific examples of an optionally substituted aromatic ring group include a group represented by Formula (I) below.

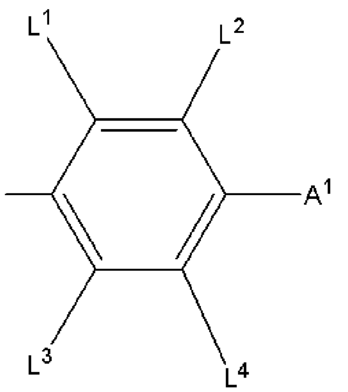

(I)

In Formula (I) above, $L^1$ to $L^4$ are identical or different, and each represents a hydrogen atom, a fluorine atom, or a lower alkyl group. Of these groups, examples of a lower alkyl group include the same lower alkyl groups as those mentioned above in relation to $Ar^1$ and $Ar^2$. $A^1$ represents a hydrogen atom, a fluorine atom, an alkoxy group, a fluoroalkyl group, a fluoroalkoxy group, or $SF_5$. Of these, examples of alkoxy, fluoroalkyl, and fluoroalkoxy groups include the same groups as those mentioned above in relation to $Ar^1$ and $Ar^2$.

The substitution positions $L^1$ to $L^4$ and $A^1$ on the benzene ring represented by Formula (I) are not limited to the positions shown in Formula (I), and may be any positions on the benzene ring.

Example of the groups represented by Formula (I) above include phenyl, 2-fluorophenyl, 3-fluorophenyl, 4-fluorophenyl, 2,3-difluorophenyl, 2,4-difluorophenyl, 2,5-difluorophenyl, 2,6-difluorophenyl, 3,4-difluorophenyl, 3,5-difluorophenyl, 2,3,4-trifluorophenyl, 2,3,5-trifluorophenyl, 2,3,6-trifluorophenyl, 2,4,5-trifluorophenyl, 2,4,6-trifluorophenyl,

3,4,5-trifluorophenyl, 4-trifluoromethylphenyl, 3,5-difluoro-4-trifluoromethylphenyl, 4-methoxyphenyl, 3,5-difluoro-4-methoxyphenyl, 4-(trifluoromethoxy)phenyl, 3,5-difluoro-4-(trifluoromethoxy)phenyl, 4-(pentafluorosulfanyl)phenyl, and the like.

Of the groups represented by $Ar^2$, specific examples of an optionally substituted aromatic ring group include a group represented by Formula (II) below.

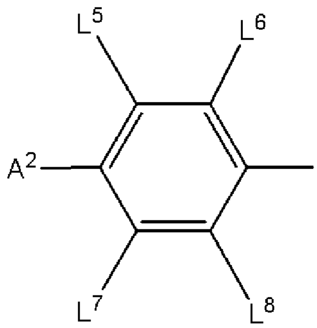

(II)

In Formula (II) above, $L^5$ to $L^8$ are identical or different, and each represents a hydrogen atom, a fluorine atom, or a lower alkyl group. Of these groups, examples of lower alkyl groups include the same lower alkyl groups as those mentioned above in relation to $Ar^1$ and $Ar^2$. $A^2$ represents a halogen atom, a lower alkyl group, a cycloalkyl group, or an aromatic ring group, and the cycloalkyl group and aromatic ring group may further optionally substituted with a lower alkyl group, an optionally substituted aromatic ring group, and the like. Of these groups, examples of a halogen atom include fluorine, chlorine, bromine, and iodine, and examples of lower alkyl groups include the same lower alkyl groups mentioned above in relation to $Ar^1$ and $Ar^2$. Examples of an aromatic ring group include phenyl, naphthyl, and the like, and examples of an optionally substituted aromatic ring group include aromatic ring groups substituted with lower alkyl, cycloalkyl, halogen, phenyl, and the lie. A phenyl group as a substituent may be further optionally substituted with halogen. The lower alkyl, cycloalkyl, and halogen in these groups are the same as those represented by $Ar^1$ and $Ar^2$.

The substitution positions $L^5$ to $L^8$ and $A^2$ on the benzene ring represented by Formula (II) above are not limited to the positions shown in Formula (II), and may be any positions on the benzene ring.

Specific examples of the groups represented by Formula (II) above include phenyl, 2,6-difluorophenyl, 4-methylphenyl, 4-ethylphenyl, 4-propylphenyl, 4-butylphenyl, 4-pentylphenyl, 4-hexylphenyl, 4-cyclobutylphenyl, 4-cyclopentylphenyl, 4-cyclohexylphenyl, 4-(4-propylcyclohexyl)phenyl, biphenyl, 3,5-difluorobiphenyl, 4'-methylbiphenyl, 4'-ethylbiphenyl, 4'-propylbiphenyl, 4'-butylbiphenyl, 4'-pentylbiphenyl, 4'-hexylbiphenyl, 4'-cyclohexylbiphenyl, terphenyl, 2,6-difluoroterphenyl, 4-chlorophenyl, 4-bromophenyl, 4-iodophenyl, 4-chloro-2-fluorophenyl, 4-bromo-2-fluorophenyl, 4-iodo-2-fluorophenyl, 4-chloro-2,6-difluorophenyl, 4-bromo-2,6-difluorophenyl, 4-iodo-2,6-difluorophenyl, 4'-chlorobiphenyl, 4'-bromobiphenyl, 4'-iodobiphenyl, 4'-chloro-3-fluorobiphenyl, 4'-bromo-3-fluorobiphenyl, 4'-iodo-3-fluorobiphenyl, 4'-chloro-3,5-difluorobiphenyl, 4'-bromo-3,5-difluorobiphenyl, 4'-iodo-3,5-difluorobiphenyl, and the like.

When an aryl ester of arylthionocarboxylic acid in which $Ar^1$ represents a group represented by Formula (I) above and $Ar^2$ represents a group represented by Formula (II) above is used as a starting material, and when, in particular, fluorination is performed with the fluorinating agent $IF_n$ (particularly preferably iodine pentafluoride) under the conditions described below, difficult-to-separate impurities are not likely to be generated, and purification is easily performed by a usual separation method, such as column chromatography and recrystallization, thus making it possible to easily obtain a high-purity target product.

In the reaction above, the fluorinating agent $IF_n$ (particularly preferably iodine pentafluoride ($IF_5$)) is used in an amount of preferably about 0.2 to 20 mol, more preferably about 0.3 to 5 mol, and still more preferably about 0.4 to 2 mol, per mol of the thionocarboxylic acid ester represented by Formula (1). The reaction temperature is not particularly limited, and is usually about -20 to 200°C, and preferably about 0 to 100°C.

The reaction time cannot be generalized because it varies according to reaction conditions. It is normally within a range of about 5 minutes to 300 hours.

A reaction solvent may be used, if required, and is preferably used. Examples of reaction solvents include pentane, hexane, heptane, cyclohexane, petroleum ether, and like aliphatic solvents, dichloromethane, dichloroethane, chloroform, fluorotrichloromethane, 1,1,2-trichlorotrifluoroethane, 2-chloro-1,2-dibromo-1,1,2-trifluoroethane, 1,2-dibromohexafluoropropane, 1,2-dibromotetrafluoroethane, 1,1-difluorotetrachloroethane, 1,2-difluorotetrachloroethane, heptafluoro-2,2,3-trichlorobutane, 1,1,1,3-tetrachlorotetrafluoropropane, 1,1,1-trichloropentafluoropropane, 1,1,1-trichlorotrifluoroethane, polychlorotrifluoroethylene, and like halogenated-aliphatic solvents, methyl formate, ethyl formate, methyl acetate, ethyl acetate, propyl acetate, butyl acetate, methyl propionate, γ-butyrolactone, propylene carbonate, and like ester solvents, acetonitrile, propionitrile, and like nitrile solvents, benzene, chlorobenzene, toluene, dichlorobenzene,

fluorobenzene, nitrobenzene, and like aromatic solvents, diethyl ether, dipropyl ether, tetrahydrofuran, 2-methyltetrahydrofuran, tetrahydropyran, 4-methyltetrahydropyran, monoglyme, diglyme, and like ether solvents, N,N-dimethylformamide (DMF), dimethylsulfoxide (DMSO), water, nitromethane, N,N-diethylformamide, N,N-dimethylacetamide, 1-methyl-2-pyrrolidone, 1,3-dimethyl-2-imidazolidinone (DMI), tetramethyl urea, 1,3-dimethylpropylene urea, hexamethylphosphoramide (HMPA), and the like. These may be used alone or in any combination of two or more.

In the present invention, in particular, the thionocarboxylic acid ester represented by Formula (1) is reacted with iodine pentafluoride, preferably in the presence of at least either one of an organic base or HF, and more preferably in the presence of both an organic base and HF. This greatly improves the yield of a compound having an oxydifluoromethylene skeleton represented by Formula (2):

$$Ar^2\text{-}CF_2\text{-}O\text{-}Ar^1 \qquad (2),$$

wherein $Ar^1$ and $Ar^2$ are as defined above.

Examples of usable organic bases include aliphatic amines (primary amines, secondary amines, tertiary amines), alicyclic amines (secondary amines, tertiary amines), aromatic amines (primary amines, secondary amines, tertiary amines), heterocyclic amines, and the like. These organic bases may be used alone or in a combination of two or more. Specific examples of these organic bases are shown below.

Examples of aliphatic primary amines include methylamine, ethylamine, propylamine, butylamine, pentylamine, hexylamine, cyclohexylamine, ethylenediamine, and the like; examples of aliphatic secondary amines include dimethylamine, diethylamine, dipropylamine, dibutylamine, dipentylamine, dihexylamine, dicyclohexylamine, and the like; examples of aliphatic tertiary amines include trimethylamine, triethylamine, diisopropylethylamine, N,N,N',N'-tetramethylethylenediamine, and the like. Examples of alicyclic secondary amines include piperidine, piperazine, pyrrolidine, and morpholine; and examples of alicyclic tertiary amines include N-methylpiperazine, N-methylpyrrolidine, 5-diazabicyclo[4.3.0]nonane-5-ene, and 1,4-diazabicyclo[2.2.2]octane. Examples of aromatic amines include aniline, methylaniline, dimethylaniline, N,N-dimethylaniline, haloaniline, nitroaniline, and the like. Examples of heterocyclic amines include pyridine, pyrimidine, piperazine, quinoline, imidazole, and the like. Examples of organic bases further include polymer-supported amine compounds, such as polyallylamine and polyvinylpyridine.

The amount of the organic base used may be selected from a range from a catalytic amount to a large excess, and is preferably about 0.5 to 5 mol, more preferably about 0.6 to 4 mol, and still more preferably about 0.8 to 3 mol, per mol of the thionocarboxylic acid ester represented by Formula (1).

The amount of HF used may be selected from a range from a catalytic amount to a large excess, and is preferably about 1 to 10 mol, and more preferably about 1 to 5 mol, per mol of the organic base.

The organic base and HF may be added separately, or may be added as a hydrofluoride salt of an organic base.

When iodine pentafluoride is used as the fluorinating agent $IF_n$, together with pyridine as a base, it is possible to use a complex of iodine pentafluoride with a solid of pyridine and hydrogen fluoride. This complex may contain $IF_5$, pyridine, HF, or a combination thereof, that do not constitute the $IF_5$-pyridine-HF complex, to an extent that the fluorination reaction is not significantly inhibited.

The above complex may be obtained by adding iodine pentafluoride ($IF_5$) to, for example, a mixture of 50 mol% pyridine and 50 mol% anhydrous hydrogen fluoride, followed by mixing. The $IF_5$-pyridine-HF complex consisting of equimolar amounts of each component is a solid and an easy-to-handle compound. Thus, the use of this complex as a fluorinating agent makes the operation of the fluorination reaction of thionocarboxylic acid ester simple, and the reaction is performed efficiently.

In the method described above, a compound having an oxydifluoromethylene skeleton represented by Formula (2):

$$Ar^2\text{-}CF_2\text{-}O\text{-}Ar^1 \qquad (2),$$

wherein $Ar^1$ and $Ar^2$ are as defined above, is obtained in high yield.

The compound having an oxydifluoromethylene skeleton obtained by the above method is optionally purified by a known method, such as quenching, extraction, drying, silica gel column chromatography, and recrystallization, to be collected.

In the above method, there is no particular limitation on how to produce a thionocarboxylic acid ester represented by Formula (1) used as a starting material, and various known methods may be used, such as a method comprising reacting a chlorothionoformic acid ester with an aromatic compound (H. Viola, et al., Chem. Ber., 101, 3517 (1968)), a method comprising treating a carbanion with a dithiocarbonic acid ester or a thionocarbonic acid ester (Liebigs Ann. Chem., 1973, 1637), a method comprising reacting an orthoester with hydrogen sulfide (A. Ohno, et al., Tetrahedron Lett., 1968, 2083), a method comprising treating a carboxylic acid ester with phosphorus pentasulfide or Lawesson's

reagent (Synthesis, 1973, 149; Bull. Chem. Soc. Belg., 87, 293 (1987)), a method comprising reacting a thionocarboxylic acid chloride with an alcohol or phenol (S. Scheithauer et al., Chem. Ber., 98, 838(1965)), a method comprising reacting a nitrile with an alcohol and then reacting the resulting product with hydrogen sulfide (Liebigs Ann. Chem., 1974, 671), a method comprising treating a methylated aromatic compound with sulfur and an alcohol (Z. Chem., 6, 108 (1966)), and a method comprising reacting a thioacyl disulfide with an alcoholate (K. A. Latif et al., Tetrahedron, 26, 4247 (1970)). In the present invention, for example, a halogenated thiocarbonyl compound used as a starting material is reacted with a compound represented by a specific formula; this method enables the production of a thionocarboxylic acid ester at a high yield by a simple production step using relatively safe starting materials. The following specifically describes this method.

(II) Production Step of Thionocarboxylic Acid Ester

In this step, a halogenated thiocarbonyl compound represented by Formula (3):

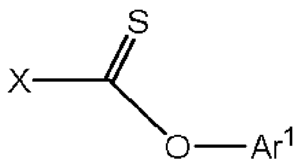

(3)

,

wherein $Ar^1$ is as defined above, and X represents a halogen atom, and
a compound represented by Formula (4):

$Ar^2$-M (4),

wherein $Ar^2$ is as defined above, and M represents an alkali metal, monovalent copper, or group: -$MgY^1$, wherein $Y^1$ represents a halogen atom,
are used as starting materials, and these compounds are reacted to give a thionocarboxylic acid ester represented by Formula (1):

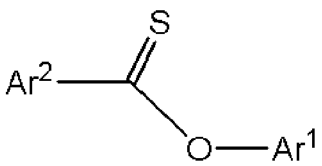

(1)

,

wherein $Ar^1$ and $Ar^2$ are as defined above.

This method enables the production of a thionocarboxylic acid ester at a high yield by a simple production step using relatively safe starting materials, and does not require the use of difficult-to-handle starting materials, such as dibromodifluoromethane and bromodifluoromethane.

The following first describes starting compounds used in this step.

(i) Halogenated Thiocarbonyl Compound

In a halogenated thiocarbonyl compound represented by Formula (3) used as a starting material:

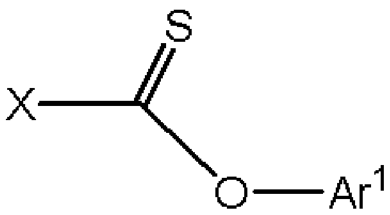

(3)

,

$Ar^1$ represents an optionally substituted aromatic ring group, an optionally substituted cycloalkyl group, or an

optionally substituted alkyl group. Specifically, $Ar^1$ here represents the same group as that represented by $Ar^1$ in Formula (1). Specific examples of $Ar^1$ in Formula (3) include the same groups as those included in the specific examples of $Ar^1$ in Formula (1).

Examples of the halogen atom represented by X in Formula (3) above include fluorine, chlorine, bromine, iodine, and the like, with chlorine being particularly preferable.

(ii) Compound of Formula (4)

Of the starting materials used in the above reaction, $Ar^2$ in Formula (4):

$$Ar^2\text{-}M \qquad (4)$$

represents an optionally substituted aromatic ring group, an optionally substituted cycloalkyl group, or an optionally substituted alkyl group. Specifically, the groups represented by $Ar^2$ are the same as those represented by $Ar^2$ in Formula (1).

In the compound of Formula (4), when M represents group: $-MgY^1$, the halogen atom as a substituent is preferably a fluorine atom, a chlorine atom, or the like.

The compound represented by Formula (4) above in which M is an alkali metal, i.e., an alkali metal compound represented by Formula (4'): $Ar^2$-$M^1$, wherein $Ar^2$ is as defined above, and $M^1$ is an alkali metal, may be obtained by reacting a halide represented by Formula (5): $Ar^2$-$Y^2$, wherein $Ar^2$ is as defined above, and $Y^2$ represents a halogen atom other than fluorine or a hydrogen atom, with an organoalkali metal compound represented by Formula (6): R-$M^1$, wherein R represents an alkyl group or a phenyl group, and $M^1$ represents an alkali metal. Examples of alkali metals represented by $M^1$ above include Li, Na, K, and the like.

Specific examples of halogen atoms other than fluorine represented by $Y^2$ in Formula (5): $Ar^2$-$Y^2$ representing a halide include chlorine, bromine, iodine, and the like.

R in Formula (6): R-$M^1$ representing an organoalkali metal compound represents an alkyl group or a phenyl group. Examples of an alkyl group include straight or branched alkyl groups having about 1 to 5 carbon atoms.

The organoalkali metal compound represented by Formula (6): R-$M^1$ is used in an amount of about 0.9 to 5.0 mol, and preferably about 0.9 to 1.5 mol, per mol of the halide represented by Formula (5): $Ar^2$-$Y^2$.

The reaction between the halide represented by Formula (5): $Ar^2$-$Y^2$ and the organoalkali metal compound represented by Formula (6): R-$M^1$ is preferably performed in an aprotic organic solvent that is inactive to the reaction. Examples of the aprotic organic solvent include tetrahydrofuran, diethyl ether, 2-methyl tetrahydrofuran, dibutyl ether, tert-butylmethyl ether, cyclopentylmethyl ether, dimethoxyethane, dioxane, benzene, toluene, xylene, pentane, hexane, heptane, and the like. These aprotic organic solvents may be used in a combination of two or more, if necessary.

The starting material concentration in the reaction solvent is not particularly limited. For example, the concentration of the halide represented by Formula (5): $Ar^2$-$Y^2$ is about 0.1 to 3 mol/L.

The reaction temperature in the reaction between the halide represented by Formula (5): $Ar^2$-$Y^2$ and the organoalkali metal compound represented by Formula (6): R-$M^1$ is preferably room temperature or lower, and more preferably about -78 to 2°C.

The pressure at the time of the reaction is not particularly limited, and the reaction may be usually performed under ordinary pressure. The atmosphere at the time of the reaction is not particularly limited, and is usually preferably an inert gas atmosphere, such as nitrogen and argon. The reaction time is not particularly limited, and is usually about 5 minutes to 10 hours.

The method described above produces an alkali metal compound represented by Formula (4'): $Ar^2$-$M^1$, wherein $Ar^2$ and $M^1$ are as defined above.

The reaction between the alkali metal compound obtained by the above method represented by Formula (4'): $Ar^2$-$M^1$ and a halogenated thiocarbonyl compound represented by Formula (3) gives a target thionocarboxylic acid ester represented by Formula (1) :

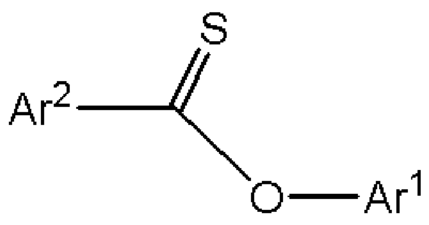

(1)

.,

wherein $Ar^1$ and $Ar^2$ are as defined above.

An alternative method comprises reacting the alkali metal compound represented by Formula (4'): $Ar^2$-$M^1$, wherein $Ar^2$ and $M^1$ are as defined above, with a monovalent copper compound to convert it into a compound represented by Formula (4"): $Ar^2$-Cu, wherein $Ar^2$ is as defined above, and then reacting the resulting compound with a halogenated thiocarbonyl compound represented by Formula (3). This method more greatly improves the yield of the thionocarboxylic acid ester represented by Formula (1).

The copper compound represented by Formula (4"): $Ar^2$-Cu may be obtained by reacting the alkali metal compound represented by Formula (4'): $Ar^2$-$M^1$ with a monovalent copper compound. Examples of the monovalent copper compound used in this reaction include copper halides, such as copper chloride, copper bromide, and copper iodide; copper cyanide (CuCN); and the like.

The monovalent copper compound is used in an amount of about 0.1 to 5 mol, and preferably about 0.7 to 1.2 mol, per mol of the alkali metal compound represented by Formula (4'): $Ar^2$-$M^1$.

The reaction between the alkali metal compound represented by Formula (4'): $Ar^2$-$M^1$ and a monovalent copper compound is preferably performed in an aprotic organic solvent. Examples of the aprotic organic solvent include the same solvents as those used in the reaction between the halide represented by Formula (5): $Ar^2$-$Y^2$ and the organoalkali metal compound represented by Formula (6): R-$M^1$.

The starting material concentration in the solvent is not particularly limited. For example, the concentration of the alkali metal compound represented by Formula (4'): $Ar^2$-$M^1$ is about 0.1 to 3 mol/L.

The reaction temperature in the reaction between the alkali metal compound represented by Formula (4'): $Ar^2$-$M^1$ and a monovalent copper compound is preferably room temperature or lower, preferably about -78°C to room temperature, and more preferably about -30 to 2°C.

The pressure at the time of the reaction is not particularly limited, and the reaction may be usually performed under ordinary pressure. The atmosphere at the time of the reaction is not particularly limited, and is usually preferably an inert gas atmosphere, such as nitrogen and argon. The reaction time is usually about 5 minutes to 10 hours.

The above method gives a metal compound represented by Formula (4): $Ar^2$-M in which M represents Cu, i.e., a copper compound represented by Formula (4"): $Ar^2$-Cu.

A compound represented by Formula (4) above in which M represents group: -Mg$Y^1$, i.e., a Grignard compound represented by Formula (4'''): $Ar^2$-Mg$Y^1$, wherein $Ar^2$ is as defined above, and $Y^1$ is a halogen atom, may be obtained by reacting a halide represented by Formula (7): $Ar^2$-$Y^1$, wherein $Ar^2$ and $Y^1$ are as defined above, with a magnesium compound. Examples of the halogen atom represented by $Y^1$ above include chlorine, bromine, iodine, and the like.

The magnesium compounds usable in this reaction are not particularly limited, as long as they can react with a halide represented by Formula (7): $Ar^2$-$Y^1$ to form a Grignard compound. Specific examples of the magnesium compounds include metal magnesium, i-PrMgCl (isopropylmagnesium chloride), i-PrMgBr (isopropylmagnesium bromide), ate complexe i-$PrBu_2MgLi$, which may be prepared from n-BuLi with i-PrMgCl or i-PrMgBr, and the like.

The magnesium compound is used in an amount of about 1 to 10 mol, and preferably about 1 to 2 mol, per mol of the halide represented by Formula (7): $Ar^2$-$Y^1$.

The reaction between the halide represented by Formula (7): $Ar^2$-$Y^1$ and a magnesium compound is usually preferably performed in an aprotic solvent that is inactive to the reaction. As the aprotic solvent, for example, the same solvents as those used in the reaction between the halide represented by Formula (5): $Ar^2$-$Y^2$ and the organoalkali metal compound represented by Formula (6): R-$M^1$ may be used.

The starting material concentration in the reaction solvent is not particularly limited. For example, the concentration of the halide represented by Formula (7): $Ar^2$-$Y^1$ is about 0.5 to 10 mol/L.

The reaction temperature in the reaction between the halide represented by Formula (7): $Ar^2$-$Y^1$ and a magnesium compound is preferably about 0 to 100°C, and more preferably about 20 to 70°C.

The pressure at the time of the reaction is not particularly limited, and the reaction may be usually performed under ordinary pressure. The atmosphere at the time of the reaction is not particularly limited, and is usually preferably an inert gas atmosphere, such as nitrogen and argon. The reaction time is usually about 30 minutes to 10 hours.

The reaction between the halide represented by Formula (7): $Ar^2$-$Y^1$ and a magnesium compound, when performed in the presence of LiCl, iodine, dibromoethane, or the like, further improves the yield of the Grignard compound represented by Formula (4'''): $Ar^2$-Mg$Y^1$. These compounds are used in an amount of preferably about 1.0 to 5.0 mol, and more preferably about 1.0 to 1.2 mol, per mol of the halide represented by Formula (7): $Ar^2$-$Y^1$.

The above method gives the Grignard compound represented by Formula (4'''): $Ar^2$-Mg$Y^1$.

(iii) Reaction Method

As described above, in the reaction step above, a halogenated thiocarbonyl compound represented by Formula (3):

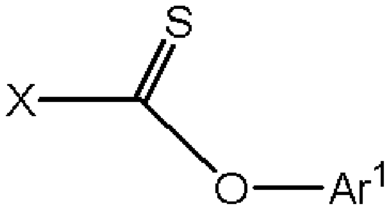

(3)

,

wherein $Ar^1$ is as defined above, and X represents a halogen atom, is reacted with a compound represented by Formula (4):

$Ar^2$-M (4),

wherein $Ar^2$ is as defined above, and M represents an alkali metal, monovalent copper, or group: $-MgY^1$.

In this manner, a thionocarboxylic acid ester represented by Formula (1):

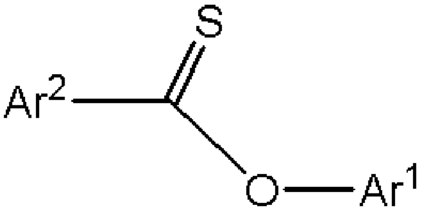

(1)

,

wherein $Ar^1$ and $Ar^2$ are as defined above, is obtained.

The compound represented by Formula (4): $Ar^2$-M is used in an amount of about 0.8 to 2 mol, and preferably about 0.9 to 1.2 mol, per mol of the halogenated thiocarbonyl compound represented by Formula (3). In particular, when M represents an alkali metal or monovalent copper, the compound represented by Formula (4): $Ar^2$-M is used in an amount of preferably about 1 to 1.5 mol, and more preferably about 1 to 1.2 mol, per mol of the halogenated thiocarbonyl compound represented by Formula (3).

In the above reaction step, in particular, the compound represented by Formula (4): $Ar^2$-M is preferably a compound represented by Formula (4) in which M represents an alkali metal, in particular Li, from the viewpoint of reducing the formation of by-products.

The reaction between the halogenated thiocarbonyl compound represented by Formula (2) and the compound represented by Formula (4): $Ar^2$-M is preferably performed in an aprotic solvent. As the aprotic organic solvent, for example, the same solvents as those used in the reaction between the halide represented by Formula (5): $Ar^2$-$Y^2$ and the organoalkali metal compound represented by Formula (6): R-$M^1$ may be used.

The starting material concentration in the reaction solvent is not particularly limited. For example, the concentration of the halogenated thiocarbonyl compound represented by Formula (3) is about 0.1 to 3 mol/L.

The reaction temperature in the reaction between the halogenated thiocarbonyl compound and the compound represented by Formula (4): $Ar^2$-M in which M represents an alkali metal or monovalent copper is preferably room temperature or lower, and more preferably about -30 to 2°C. When M in Formula (4): $Ar^2$-M represents group: $-MgY^1$, then the reaction temperature is preferably about -78°C to room temperature, and more preferably about -50 to 0°C.

The pressure at the time of the reaction is not particularly limited, and the reaction may be usually performed under ordinary pressure. The atmosphere at the time of the reaction is not particularly limited, and is usually preferably an inert gas atmosphere, such as nitrogen and argon. The reaction time is usually about 5 to 10 hours.

The above method produces a high yield of a thionocarboxylic acid ester represented by Formula (1):

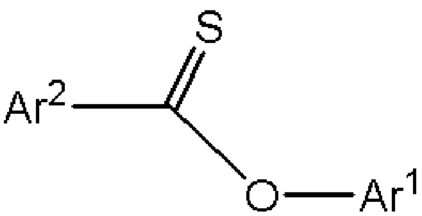

(1)

,

wherein $Ar^1$ and $Ar^2$ are as defined above.

In particular, when the compound represented by Formula (4): $Ar^2$-M in which M is Cu, i.e., a copper compound

represented by Formula (4"): $Ar^2$-Cu, is used, the thionocarboxylic acid ester represented by Formula (1) is produced at a particularly high yield.

In the above reaction step, in particular, when the reaction between the halogenated thiocarbonyl compound represented by Formula (3) and the compound represented by Formula (4): $Ar^2$-M is performed in the presence of a catalyst, a more improved yield of the thionocarboxylic acid ester represented by Formula (1) is achieved. In particular, when the compound represented by Formula (4): $Ar^2$-M is the alkali metal compound represented by Formula (4'): $Ar^2$-$M^1$, a greatly improved yield of the thionocarboxylic acid ester represented by Formula (1) is achieved.

When M in Formula (4): $Ar^2$-M represents an alkali metal or monovalent copper, it is possible to use a catalyst, such as a trivalent iron compound and a divalent nickel compound. These compounds may be used alone or in a combination of two or more. Specific examples of trivalent iron compounds include tris(2,4-pentanedionato)iron(III), ferric chloride, and the like, and specific examples of divalent nickel compounds include nickel chloride and the like. The catalyst is used in an amount of preferably about 0.03 mol or more, per mol of the halogenated thiocarbonyl compound represented by Formula (3). Although the upper limit of the amount of the catalyst used is not particularly limited, an excess amount would not achieve further improvement in the effect and would be disadvantageous in cost. The amount is thus preferably about 0.5 mol or less, and more preferably about 0.1 mol or less, per mol of the halogenated thiocarbonyl compound.

When M in Formula (4): $Ar^2$-M represents group: -$MgY^1$, a catalyst, such as a trivalent iron compound, a monovalent or divalent copper compound, a divalent nickel compound, a divalent zinc compound, and a divalent cobalt compound, may be used. These compounds may be used alone or in a combination of two or more. Specific examples of trivalent iron compounds include tris(2,4-pentanedionato)iron(III), ferric chloride, and the like. Specific examples of monovalent or divalent copper compounds include copper halides, such as copper chloride, copper bromide, and copper iodide; and copper cyanide (CuCN), cupric chloride, tetrachlorocopper(II) dilithium ($Li_2CuCL_4$), and the like. Specific examples of divalent nickel compounds include nickel chloride and the like. Specific examples of divalent zinc compounds include zinc chloride and the like. Specific examples of divalent cobalt compounds include cobalt chloride and the like. These catalysts are used in an amount of preferably about 0.01 to 2.00 mol, and more preferably about 0.01 to 0.10 mol, per mol of the halogenated thiocarbonyl compound represented by Formula (3).

In the present invention, the above method comprising directly reacting a halogenated thiocarbonyl compound represented by Formula (3):

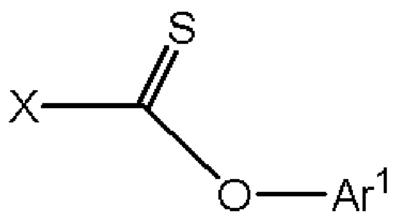

(3)

,

wherein $Ar^1$ is as defined above, and X represents a halogen atom, with a compound represented by Formula (4):

$Ar^2$-M (4),

wherein $Ar^2$ is as defined above, and M represents an alkali metal, monovalent copper, or group: -MgY, enables the production of a thionocarboxylic acid ester represented by Formula (1):

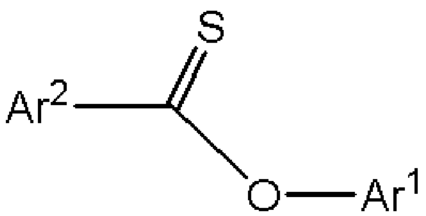

(1)

,

wherein $Ar^1$ and $Ar^2$ are as defined above.

However, this method can be replaced with a method that uses, as a starting material, a halide represented by Formula (5): $Ar^2$-$Y^2$ or Formula (7): $Ar^2$-$Y^1$, which is a starting material of the compound represented by Formula (4), and performs successive reactions to obtain the thionocarboxylic acid ester represented by Formula (1).

For example, when M in Formula (4): $Ar^2$-M represents an alkali metal or monovalent copper, the halide represented by Formula (5): $Ar^2$-$Y^2$ and the organoalkali metal compound represented by Formula (6): R-$M^1$, which are

used as starting materials of the metal compound represented by Formula (4): $Ar^2$-M, are reacted, and then, the obtained reaction product is reacted with a monovalent copper compound, if necessary. Subsequently, a halogenated thiocarbonyl compound represented by Formula (3):

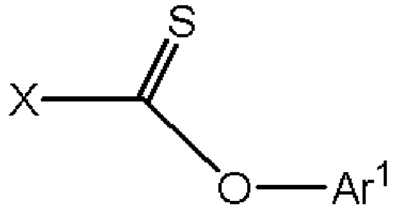

(3)

,

wherein $Ar^1$ represents an optionally substituted aromatic ring group, and X represents a halogen atom, is added thereto to cause a reaction. This method enables the production of a thionocarboxylic acid ester represented by Formula (1):

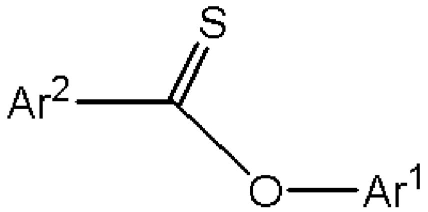

(1)

wherein $Ar^1$ and $Ar^2$ are as defined above.

Further, when M in Formula (4): $Ar^2$-M represents group: $-MgY^1$, the halide represented by Formula (7): $Ar^2$-$Y^1$ and a magnesium compound used as starting materials of a Grignard compound represented by Formula (4'''): $Ar^2$-$MgY^1$ may be reacted, followed by a reaction of the reaction product with a halogenated thiocarbonyl compound represented by Formula (3):

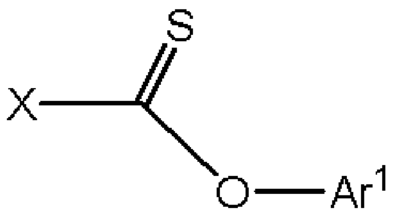

(3)

wherein $Ar^1$ and X are as defined above. This method also gives a thionocarboxylic acid ester represented by Formula (1):

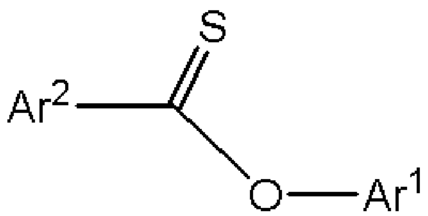

(1)

wherein $Ar^1$ and $Ar^2$ are as defined above.

In these methods, intermediate products, such as an alkali metal compound represented by Formula (4'): $Ar^2$-$M^1$, a copper compound represented by Formula (4"): $Ar^2$-Cu, and a Grignard compound represented by Formula (4'''): $Ar^2$-MgY, do not need to be separated, and a target thionocarboxylic acid ester represented by Formula (1) is obtained at a high yield by a successive process. The reaction conditions in each reaction step of this method may be the same as the reaction conditions of each reaction step described above.

The thionocarboxylic acid ester obtained by the above method may be optionally purified by a known method, such as silica gel column chromatography and recrystallization, to be collected.

When the thionocarboxylic acid ester of Formula (1) obtained by the above method is further subjected to a fluorination reaction step using the fluorinating agent $IF_n$ (particularly preferably iodine pentafluoride ($IF_5$)) as a fluorinating

agent, a compound having an oxydifluoromethylene skeleton represented by Formula (2) is obtained at a high yield while generating almost no difficult-to-separate impurities.

Therefore, when the production step of thionocarboxylic acid ester is performed in combination with the fluorination reaction step using the fluorinating agent $IF_n$ (particularly preferably iodine pentafluoride ($IF_5$)) as a fluorinating agent, a compound having an oxydifluoromethylene skeleton is obtained at a high yield by an industrially advantageous method using relatively highly safe starting materials.

The compound having an oxydifluoromethylene skeleton represented by Formula (2) obtained by the above method:

$$Ar^2\text{-}Z\text{-}Ar^1 \qquad (2),$$

wherein $Ar^1$, $Ar^2$, and Z are as defined above,
is useful as a liquid crystal material, a medicinal drug, an intermediate thereof, and the like.

Moreover, when $Ar^1$ or $Ar^2$ in Formula (2) represents a group or atom having reactivity with a boronic acid compound, or represents a group substituted with a boronic acid group, and when the compound of Formula (2) is further reacted with a boronic acid compound or a compound containing a group or atom having reactivity with a boronic acid compound, a coupling reaction occurs, making it possible to further introduce an other group such as an optionally substituted aromatic ring group into the compound of Formula (2) above. The compounds obtained by this method are various compounds useful as, for example, liquid crystal materials. The following specifically describes this reaction.

(III) Coupling Reaction Step

As a starting material, this reaction step uses a compound represented by Formula (2) in which one of $Ar^1$ and $Ar^2$ represents a group substituted with a group or atom having reactivity with a boronic acid compound, or represents a group substituted with a boronic acid group, from among the compounds represented by Formula (2) obtained in the step of fluorination reaction of thionocarboxylic acid ester. The following describes the starting materials used in this reaction step.

(i) Compound Having an Oxydifluoromethylene Skeleton

The compound having an oxydifluoromethylene skeleton used as a starting material may be a compound represented by Formula (2-1):

$$Ar^3\text{-}CF_2\text{-}O\text{-}Ar^4 \qquad (2\text{-}1).$$

In Formula (2-1), one of $Ar^3$ and $Ar^4$ represents a group substituted with a group or atom having reactivity with a boronic acid compound, or represents a group substituted with a boronic acid group, and the other represents an optionally substituted aromatic ring group, an optionally substituted cycloalkyl group, or an optionally substituted alkyl group.

Of these groups, a group substituted with a group or atom having reactivity with a boronic acid compound may be an aromatic ring group, cycloalkyl group, or alkyl group that are substituted with a group or atom having reactivity with a boronic acid compound, among the groups represented by $Ar^1$ and $Ar^2$ in Formula (2). Specific examples of a group or atom having reactivity with a boronic acid compound include halogen atoms, such as chlorine, bromine, and iodine.

Among the groups represented by $Ar^1$ and $Ar^2$ in Formula (2), the group substituted with a boronic acid group may be an aromatic ring group, cycloalkyl group, or alkyl group that contains a boronic acid group represented by Formula (a) below:

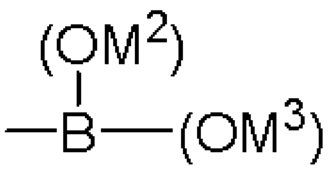

(a)

.,

as a substituent.

When a compound substituted with a boronic acid group is used as a starting material, the compound may be sometimes partly dehydration-condensed. In the present invention, it is possible to use a compound in such a condensed state as a starting material.

The above group or atom having reactivity with a boronic acid compound, and the boronic acid group may be present as a substituent directly on an aromatic ring group, cycloalkyl group, or an alkyl group, or may be attached to a substituent on these groups.

The other in the $Ar^3$ and $Ar^4$ pair represents an optionally substituted aromatic ring group, an optionally substituted cycloalkyl group, or an optionally substituted alkyl group. These groups may be those that are substituted with neither a group nor atom having reactivity with a boronic acid compound, nor a boronic acid group, from among the groups represented by $Ar^1$ or $Ar^2$ in Formula (2).

The compound represented by Formula (2-1) above is preferably a compound in which one of $Ar^3$ and $Ar^4$ is a group substituted with a group or atom having reactivity with a boronic acid compound, and particularly preferably a compound in which $Ar^3$ is a group substituted with a group or atom having reactivity with a boronic acid compound.

The compound represented by Formula (2-1) above may be obtained by using, as a starting material, a compound in which $Ar^2$ is $Ar^3$, and $Ar^1$ is $Ar^4$, i.e., a thionocarboxylic acid ester represented by Formula (1-1):

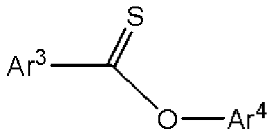

(1-1)

wherein $Ar^3$ and $Ar^4$ are as defined above, from among the compounds represented by Formula (1), and subjecting it to fluorination with the fluorinating agent $IF_n$ (particularly preferably iodine pentafluoride), as in the method described above. Further, the thionocarboxylic acid ester represented by Formula (1-1) above may be obtained by using, as a starting material, a compound represented by the formula (4) in which $Ar^2$ is $Ar^3$, i.e., a compound represented by Formula (4-1):

$Ar^3$-M (4-1),

wherein $Ar^3$ is as defined above, and M represents an alkali metal, monovalent copper, or group: $-MgY^1$, and reacting it with a halogenated thiocarboxylic acid compound represented by Formula (3-1):

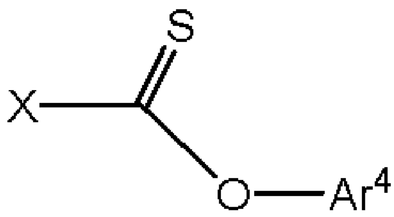

(3-1)

,

wherein $Ar^4$ is as defined above, and X represents a halogen atom, as in the method described above.

As a specific example of the compound represented by Formula (2-1) above, a compound represented by Formula (2a) below:

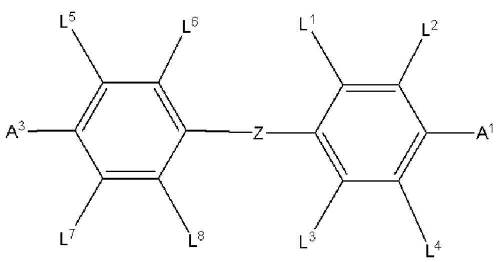

(2a)

.

can be examplified.

In Formula (2a), Z represents group: $-CF_2-O-$. $L^1$ to $L^8$ are identical or different, and each represents a hydrogen

atom, a fluorine atom, or a lower alkyl group. Of these groups, examples of a lower alkyl group include the same lower alkyl groups mentioned above in relation to $Ar^1$ and $Ar^2$.

$A^1$ represents a hydrogen atom, a fluorine atom, an alkoxy group, a fluoroalkyl group, a fluoroalkoxy group, or $SF_5$. Of these, examples of an alkoxy group include the same fluoroalkyl groups described above in relation to $Ar^1$ and $Ar^2$. Examples of a fluoroalkyl group include the same fluoroalkyl groups described above in relation to $Ar^1$ and $Ar^2$, with $CF_3$ being preferable. Examples of a fluoroalkoxy group include the same fluoroalkoxy groups described above in relation to $Ar^1$ and $Ar^2$, with $-OCF_3$ being preferable.

$A^3$ represents a halogen atom other than fluorine, or a lower alkyl group, cycloalkyl group, or phenyl group that are substituted with a halogen atom other than fluorine.

The substitution positions $L^1$ to $L^4$ and $A^1$ and $L^5$ to $L^8$ and $A^3$ on each benzene ring represented by Formula (2a) above are not limited to the positions shown in Formula (2a), and may be any positions on the benzene ring.

In Formula (2a) above, specific examples of a group represented by the following formula:

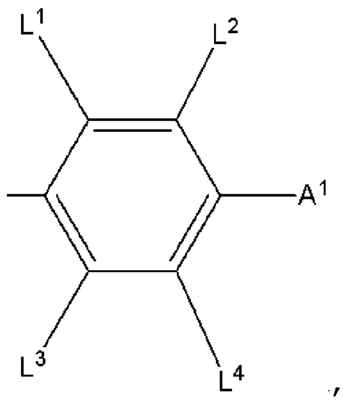

which is a group corresponding to $Ar^4$ in Formula (2-1), include the same groups that are included in the specific examples of the group represented by Formula (I) above.

Further, in Formula (2a) above, specific examples of a group represented by the following formula:

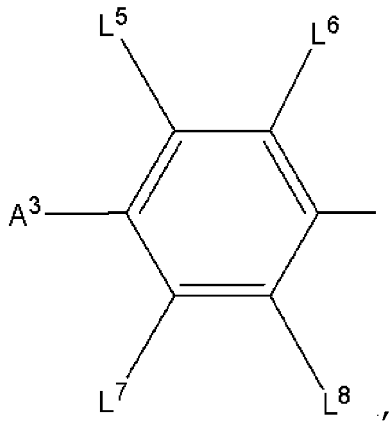

which represents a group corresponding to $Ar^3$ in Formula (2-1), include 4-chlorophenyl, 4-bromophenyl, 4-iodophenyl, 4-chloro-2-fluorophenyl, 4-bromo-2-fluorophenyl, 4-iodo-2-fluorophenyl, 4-chloro-2,6-difluorophenyl, 4-bromo-2,6-difluorophenyl, 4-iodo-2,6-difluorophenyl, 4'-chlorobiphenyl, 4'-bromobiphenyl, 4'-iodobiphenyl, 4'-chloro-3-fluorobiphenyl, 4'-bromo-3-fluorobiphenyl, 4'-iodo-3-fluorobiphenyl, 4'-chloro-3,5-difluorobiphenyl, 4'-bromo-3,5-difluorobiphenyl, 4'-iodo-3,5-difluorobiphenyl, and the like.

Among the compounds represented by Formula (8) below, the compound represented by Formula (2a) above is a compound containing a group or atom having reactivity with a boronic acid compound, and when this compound is reacted with a boronic acid compound under the conditions mentioned later, a coupling reaction occurs, making it possible to introduce the aromatic ring group or other groups contained in the boronic acid compound.

The compound represented by Formula (2a) above may be obtained by using an arylthionocarboxylic acid aryl ester represented by Formula (1a) below:

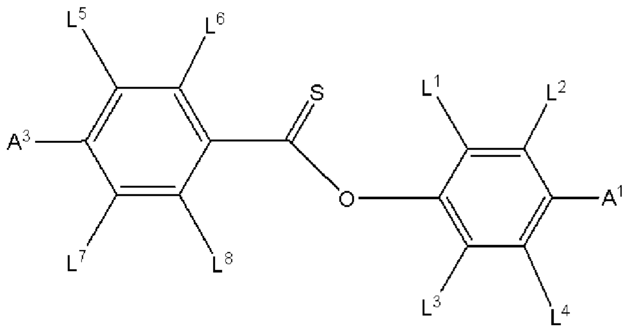

(1a)

,

as a starting material, and subjecting it to fluorination with the fluorinating agent $IF_n$ (particularly preferably iodine pentafluoride ($IF_5$)). $L^1$ to $L^8$, $A^1$, and $A^3$ in Formula (1a) above are as defined above. The reaction between the compound of Formula (1a) and iodine pentafluoride is performed under the same conditions as in the reaction described above between the thionocarboxylic acid ester of Formula (1) and iodine pentafluoride.

The arylthionocarboxylic acid aryl ester represented by Formula (1a) may be obtained by reacting a halogenated thiocarbonyl compound represented by Formula (3a):

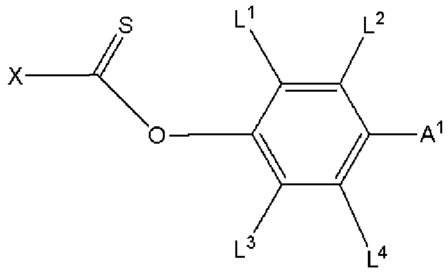

(3a)

wherein $L^1$ to $L^4$, and $A^1$ are as defined above, and X is a halogen atom,
with an aryl compound represented by Formula (4a):

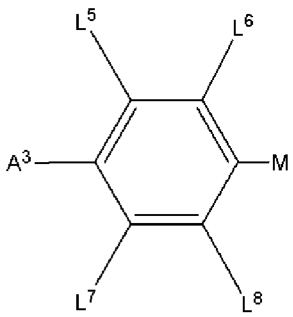

(4a)

wherein $L^5$ to $L^8$, and $A^3$ are as defined above, and M is an alkali metal, a monovalent copper, or group: $-MgY^1$ ($Y^1$ is a halogen atom).

The halogen atom represented by X in Formula (3a) is the same as that of Formula (3), and M in Formula (4a) is the same as that of Formula (4).

The reaction between the halogenated thiocarbonyl compound represented by Formula (3a) and the aryl compound represented by Formula (4a) may be performed in a similar manner as in the reaction between the halogenated thiocarbonyl compound represented by Formula (3) and the compound represented by Formula (4).

Among the aryl compounds represented by Formula (4a), an aryl compound in which M is an alkali metal, i.e., an arylated alkali metal compound represented by Formula (4a'):

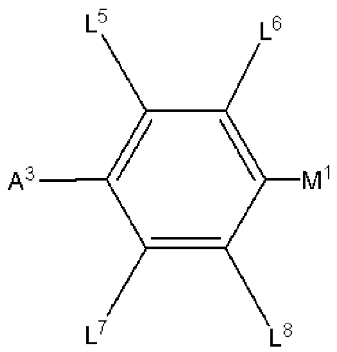

(4a')

wherein $L^5$ to $L^8$, and $A^3$ are as defined above, and $M^1$ is an alkali metal, may be obtained by reacting a halogenated aryl compound represented by Formula (5a):

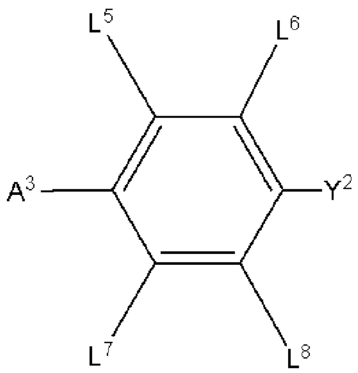

(5a)

wherein $L^5$ to $L^8$ and $A^3$ are as defined above, and $Y^2$ is a halogen atoms other than F, or a hydrogen atom, with an organoalkali metal compound represented by Formula (6):

$R-M^1$,

wherein R represents an alkyl group or a phenyl group, and $M^1$ is an alkali metal. $Y^2$ in Formula (5a) is the same as that of $Y^2$ in Formula (5) above.

The reaction between the halogenated aryl compound represented by Formula (5a) and the organoalkali metal compound represented by Formula (6) may be performed in a similar manner as in the reaction between the halide represented by Formula (5) and the organoalkali metal compound represented by Formula (6).

Further, among the aryl compounds represented by Formula (4a), the aryl compound in which M is monovalent copper, i.e., an arylated copper compound represented by Formula (4a"):

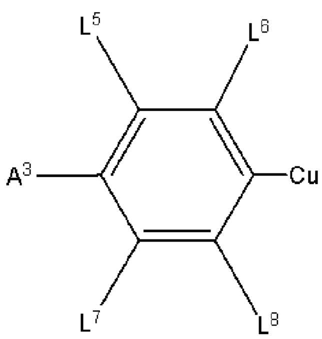

(4a")

wherein $L^5$ to $L^8$ and $A^3$ are as defined above, may be obtained by reacting an arylated alkali metal compound represented by Formula (4a'):

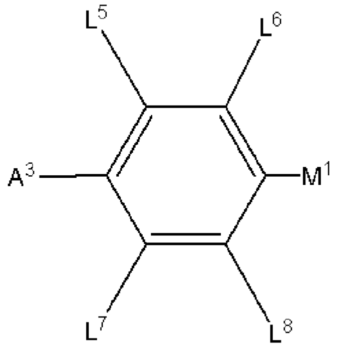

(4a')

wherein $L^5$ to $L^8$, $A^3$, and $M^1$ are as defined above, with a monovalent copper compound.

The reaction between the arylated alkali metal compound represented by Formula (4a') and a monovalent copper compound is performed in a similar manner as in the reaction described above between the arylated alkali metal compound represented by Formula (4'): $Ar^2$-$M^1$ and a monovalent copper compound.

An aryl compound represented by Formula (4a) in which M represents group: -$MgY^1$, i.e., an arylated Grignard compound represented by Formula (4a'''):

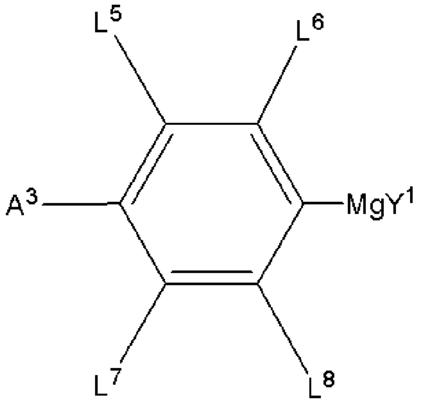

(4a''')

,

wherein $L^5$ to $L^8$ and $A^3$ are as defined above, and $Y^1$ is a halogen atom,
may be obtained by reacting a halogenated aryl compound represented by Formula (7a):

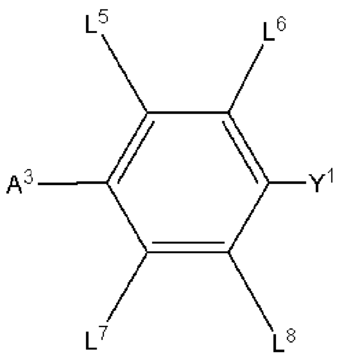

(7a)

,

wherein $A^3$, $L^5$ to $L^8$, and $Y^1$ are defined above,
with a magnesium compound.

The reaction between the halogenated aryl compound represented by Formula (7a) and a magnesium compound is performed in a similar manner as in the reaction between the halide represented by Formula (7): $Ar^2$-$Y^1$ and a magnesium compound.

(ii) Compound of Formula (8)

A compound represented by Formula (2-1):

$$Ar^3\text{-}CF_2\text{-}O\text{-}Ar^4 \qquad (2\text{-}1)$$

is subjected to a coupling reaction with a compound represented by Formula (8):

$$Ar^5\text{-}D \qquad (8).$$

D in Formula (8) represents a boronic acid group when the compound represented by Formula (2-1) is a compound containing a group or atom having reactivity with a boronic acid compound. D in Formula (8) represents a group having reactivity with a boronic acid compound when the compound represented by Formula (2-1) is a compound containing a boronic acid group.

In the above, as the boronic acid group, the same groups as those represented by formula (a):

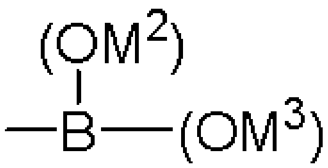

(a)

can be exemplified.

In Formula (8) above, when D represents a group or atom having reactivity with a boronic acid compound, specific examples thereof include halogen atoms, such as chlorine, bromine, and iodine.

Of the compounds represented by Formula (8), when a compound containing a boronic acid compound contains a boronic acid group represented by Formula (a), the compound becomes a boronic acid, a boronic acid ester, or a boronic acid salt.

$Ar^5$ in Formula (8) represents an optionally substituted aromatic ring group, an optionally substituted cycloalkyl group, or an optionally substituted alkyl group. Examples of the aromatic ring group, cycloalkyl group, and alkyl group in the optionally substituted aromatic ring group, optionally substituted cycloalkyl group, and optionally substituted alkyl group include the same groups as those mentioned above as represented by $Ar^1$ in Formula (1). Substituents for these groups are not particularly limited as long as it is inactive to a reaction with the compound having an oxydifluoromethylene skeleton represented by Formula (2-1). Specific examples of such a substituent include fluorine, alkyl, optionally substituted cyclic aliphatic hydrocarbon optionally containing a heteroatom, and optionally substituted phenyl. These groups are inactive to a reaction with the compound having an oxydifluoromethylene skeleton represented by Formula (2-1), and play little part in the reaction.

Specific examples of the alkyl groups as a substituent include the same alkyl groups mentioned above in relation to $Ar^1$ and $Ar^2$. Examples of the optionally substituted cyclic aliphatic hydrocarbon optionally containing a heteroatom include cycloalkyl groups having 4 to 6 carbon atoms, such as cyclohexyl, cyclopentyl, and cyclobutyl; and 4- to 6-membered cyclic aliphatic hydrocarbon groups, such as heterocyclic hydrocarbon groups in which one or more carbon atoms of these cycloalkyl groups are replaced with a heteroatom, such as oxygen. Examples of groups that can serve as a substituent for the cyclic aliphatic hydrocarbon group or phenyl group include fluorine, lower alkyl, and the like. Here, examples of the lower alkyl as a substituent for the cyclic aliphatic hydrocarbon group or phenyl group also include the same lower alkyl groups mentioned above. One or more of these substituents may be attached at any positions on a cyclic aliphatic hydrocarbon group or a phenyl group.

Of the compounds represented by Formula (8) above, the boronic acid compound may be a dehydration-condensed compound, for example, a cyclic trimer. This cyclic trimer is called a "boroxine compound" and is represented by the following chemical formula.

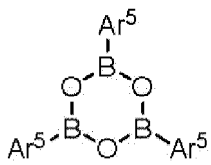

Of the compounds represented by Formula (8), as a specific example of the boronic acid compound, a compound represented by Formula (8a).

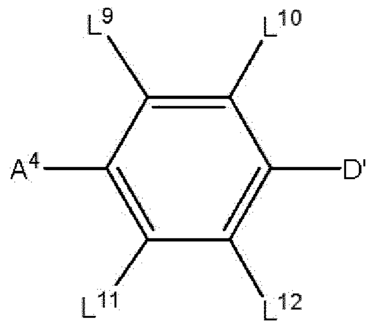

(8a)

can be exemplified.

In Formula (8a) above, $L^9$ to $L^{12}$ are identical or different, and each represents a hydrogen atom or a fluorine atom. $A^4$ represents a hydrogen atom, a fluorine atom, a lower alkyl group, an optionally substituted cycloalkyl group, or an optionally substituted phenyl group. D' represents a boronic acid group from among those represented by D in Formula (8) above.

Examples of a lower alkyl group in Formula (8a) above include the same alkyl groups as substituents in Formula (8). Examples of the optionally substituted cycloalkyl groups and optionally substituted phenyl groups in Formula (8a) include the same groups as those shown as substituents in Formula (8).

The substitution positions at $L^9$ to $L^{12}$ and $A^4$, which are the substituents on benzene ring in Formula (8a) above, are not limited to the positions shown in Formula (8a), and may be any positions on the benzene ring. The boronic acid compound represented by Formula (8a) above is particularly preferably a compound in which $A^4$ is substituted at the para-position relative to D'.

Specific examples of a boronic acid compound represented by Formula (8a) above include phenylboronic acid, 4-fluorophenylboronic acid, 4-methylphenylboronic acid, 4-ethylphenylboronic acid, 4-propylphenylboronic acid, 4-butylphenylboronic acid, 4-phenylphenylboronic acid, 4-hexylphenylboronic acid, 4-cyclohexylboronic acid, 2-fluoro-4-propylphenylboronic acid, 3-fluoro-4-propylphenylboronic acid, 2,3-difluoro-4-propylphenylboronic acid, 4-(4-fluorocyclohexyl)-phenylboronic acid, 4-(4-propylcyclohexyl)phenylboronic acid, 4'-methyl biphenylboronic acid, 4'-ethyl biphenylboronic acid, 4'-propyl biphenylboronic acid, 4'-butyl biphenylboronic acid, 4'-phenyl biphenylboronic acid, 4'-hexyl biphenylboronic acid, 2-fluoro-4'-propyl biphenylboronic acid, 3-fluoro-4'-propyl biphenylboronic acid, 2-fluoro-4'-pentyl biphenylboronic acid, 3-fluoro-4'-pentyl biphenylboronic acid, and the like.

A cyclic trimer in which the boronic acid compound represented by Formula (8a) above is dehydration-condensed is represented by the following chemical formula.

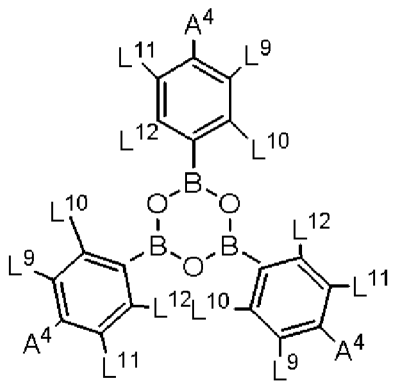

(iii) Reaction Method

In the present invention, a compound having an oxydifluoromethylene skeleton represented by Formula (2-1):

$$Ar^3\text{-}CF_2\text{-}O\text{-}Ar^4 \qquad (2\text{-}1),$$

wherein $Ar^3$ and $Ar^4$ are as defined above,
is reacted with a compound represented by Formula (8):

$Ar^5$-D (8),

wherein $Ar^5$ and D are as defined above.

This allows a coupling reaction to occur to give a compound having an oxydifluoromethylene skeleton represented by Formula(9), as defined in claim 3. Embodiments of formula (9) can be represented by formulae (9-1) and (9-2):

$Ar^5$-$Ar^6$-Z-$Ar^7$ (9-1),

wherein $Ar^5$ and Z are as defined above, $Ar^6$ represents an optionally substituted divalent hydrocarbon group, and $Ar^7$ represents an optionally substituted aromatic ring group, an optionally substituted cycloalkyl group, or an optionally substituted alkyl group,

$Ar^8$-Z-$Ar^9$-$Ar^5$ (9-2),

wherein $Ar^5$ and Z are as defined above, $Ar^9$ represents an optionally substituted divalent hydrocarbon group, and $Ar^8$ represents an optionally substituted aromatic ring group, an optionally substituted cycloalkyl group, or an optionally substituted alkyl group.

In Formulas (9-1) and (9-2) above, the optionally substituted divalent hydrocarbon group represented by $Ar^6$ and $Ar^9$ is a divalent group in which the group or atom having reactivity with a boronic acid group, or the boronic acid group is removed from a group containing a group or atom having reactivity with a boronic acid compound, or from a group containing a boronic acid group, respectively, from among the groups represented by $Ar^3$ and $A^4$ in Formula (2-1). Specific examples include optionally substituted arylene, optionally substituted cycloalkylene, or optionally substituted alkylene. The groups represented by $Ar^7$ correspond to an optionally substituted aromatic ring group, an optionally substituted cycloalkyl group, or an optionally substituted alkyl group, from among the groups represented by $Ar^3$ or $Ar^4$ in Formula (2-1).

This reaction gives a high yield of a compound having an oxydifluoromethylene skeleton using relatively inexpensive starting materials without using expensive additives and the like.

In the reaction above, the compound represented by Formula (8) is used in an amount of preferably about 0.5 to 2.0 mol, and more preferably about 0.8 to 1.2 mol, per mol of the compound having an oxydifluoromethylene skeleton represented by Formula (2-1).

The reaction above may be performed using a solvent, if necessary. As a solvent, it is preferable to use an aromatic compound, aliphatic hydrocarbon, cycloaliphatic hydrocarbon, aliphatic ether compound, cyclic ether compound, aprotic polar solvent, and protic solvent. Of these, examples of aromatic compounds include benzene, toluene, chlorobenzene, bromobenzene, and the like. Examples of aliphatic hydrocarbons include hexane, heptane, and the like. Examples of cycloaliphatic hydrocarbons include cyclohexane, and the like. Examples of aliphatic ether compounds include diethyl ether, DMG, methyl-tert-butyl ether, diethylene glycol dimethyl ether, and the like. Examples of cyclic ether compounds include tetrahydrofuran (THF), dioxane, and the like. Examples of aprotic polar solvents include dimethylformamide (DMF), dimethylsulfoxide (DMSO), acetonitrile, dimethylacetamide (DMAc), and the like. These solvents may be used alone or in a combination of two or more.

The amount of the solvent used is not particularly limited, and is usually about 0 to 20-fold mass, based on the compound having an oxydifluoromethylene skeleton represented by Formula (2-1).

The reaction temperature is also not particularly limited and is usually preferably within a range of 10 to 200°C.

The coupling reaction mentioned above is preferably performed under basic conditions. The base is preferably an alkali metal hydride, an alkali metal hydroxide, an alkali metal carbonate, or an alkali metal alkoxide. The base is preferably used in an amount of preferably about 0.8 to 4 mol, per mol of at least one aromatic boronic acid compound selected from the group consisting of the boronic acid compounds represented by Formula (8) and cyclic trimers in which a boronic acid compound represented by Formula (8) is dehydration-condensed.

The coupling reaction mentioned above is preferably performed in the presence of a catalyst. The catalyst is preferably a simple substance of a transition metal atom, or those containing these simple substances. In particular, for example, metal simple substances, such as nickel, palladium, and platinum, and alloys containing at least one of these metal simple substances are preferable. These metals on activated charcoal may also be used. The catalyst is used in an amount of preferably about 0.0005 to 0.2 mol, per mol of at least one aromatic boronic acid compound selected from the group consisting of the boronic acid compounds represented by Formula (8) and cyclic trimers in which a boronic acid compound represented by Formula (8) is dehydration-condensed.

With use of the compound represented by Formula (2-1), the above reaction gives a high yield of a compound

having an oxydifluoromethylene skeleton represented by Formula (9-1) below:

$Ar^5$-$Ar^6$-Z-$Ar^7$ (9-1),

wherein $Ar^5$, $Ar^6$, $Ar^7$, and Z are as defined above,
or
a compound having an oxydifluoromethylene skeleton represented by Formula (9-2) below:

$Ar^8$-Z-$Ar^9$-$Ar^5$ (9-2),

wherein $Ar^5$, $Ar^8$, $Ar^9$, and Z are as defined above.

In the present invention, it is preferable to use, in particular, a compound of Formula (9-1) obtained by using, as a starting material, a compound of Formula (2-1) in which $Ar^3$ represents a group substituted with a group or atom having reactivity with a boronic acid compound, or a group substituted with a boronic acid group.

In the reaction above, when at least one aromatic boronic acid compound selected from the group consisting of an aryl compound having an oxydifluoromethylene skeleton represented by Formula (2a):

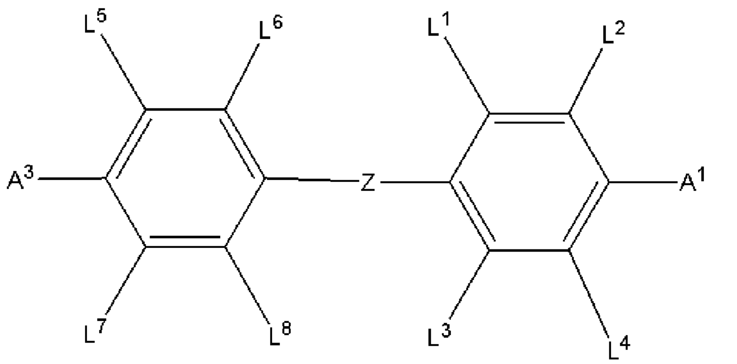

(2a)

,

wherein Z, $L^1$ to $L^8$, $A^1$, and $A^3$ are as defined above, a boronic acid compound represented by Formula (8a):

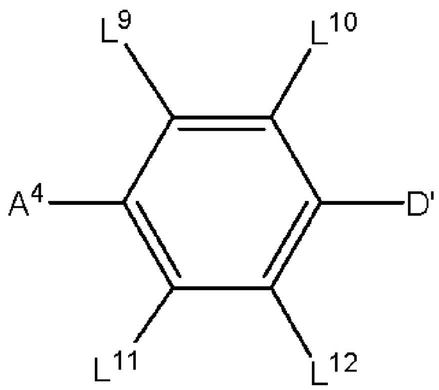

(8a)

,

wherein Z, $L^9$ to $L^{12}$, $A^4$, and D' are as defined above, and cyclic trimers in which a boronic acid compound represented by Formula (8a) is dehydration-condensed,
is used as a starting material, a high yield of a biaryl compound having an oxydifluoromethylene skeleton represented by Formula (9a) below:

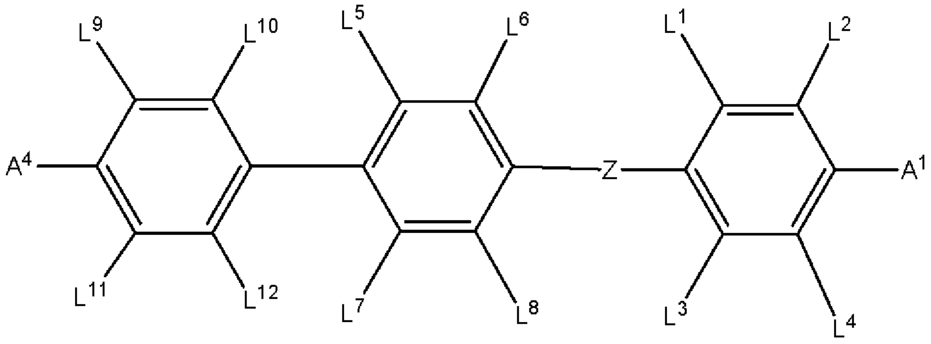

(9a)

,

wherein Z, $L^1$ to $L^{12}$, $A^1$, and $A^4$ are as defined above, is obtained.

Among the compounds represented by Formula (2a) above, a compound in which $A^3$ represents a halogen atom (Br, Cl, or I) may be converted into a compound containing a boronic acid group as a substituent by replacing the halogen atom with a boronic acid compound. This compound may be converted into a corresponding compound represented by Formula (9-1): $Ar^5$-$Ar^6$-Z-$Ar^7$ by reacting with, for example, a compound represented by Formula (8) in which D represents a group or atom having reactivity with a boronic acid compound.

The biaryl compound having an oxydifluoromethylene skeleton obtained by the above method is optionally purified by a known method, such as silica gel column chromatography and recrystallization, to be collected.

Advantageous Effects of Invention

In the present invention, a thionocarboxylic acid ester is used as a starting material and subjected to a reaction with iodine pentafluoride, the method of which enables the production of a high yield of a high-purity compound having an oxydifluoromethylene skeleton while inhibiting the generation of difficult-to-separate impurities.

Further, the thionocarboxylic acid ester used in this method may be obtained at a high yield by reacting a halogenated thiocarbonyl compound with a specific compound with the use of relatively safe starting materials under mild reaction conditions. Therefore, a combination of this reaction with the above fluorination reaction performed with iodine pentafluoride makes it possible to produce a high yield of a compound having an oxydifluoromethylene skeleton by a relatively simple reaction and purification step using highly safe starting materials.

Further, when the compound having an oxydifluoromethylene skeleton obtained by the above method is reacted with a specific boronic acid compound or a compound containing a group having reactivity with a boronic acid compound, various groups such as an aromatic ring group may be introduced into the compound having an oxydifluoromethylene skeleton.

Thus, when above reaction steps are appropriately combined and performed, various compounds having an oxydifluoromethylene skeleton useful as, for example, liquid crystal materials, medicines, and intermediates thereof are obtained with a high purity at a high yield by a simple process using relatively inexpensive, safe starting materials.

Description of Embodiments

The following describes the present invention in more detail with reference to Examples and Reference Examples.

Example 1

3,5-Difluoro-4'-propylbiphenyl-4-thionocarboxylic acid 3,4,5-trifluorophenyl ester (1.65 g, 3.9 mmol) was placed in a fluororesin container, ethyl acetate (10 mL) was added thereto, and iodine pentafluoride ($IF_5$) (0.86 g, 3.9 mmol) and triethylamine-trihydrofluoride salt ($NEt_3$-3HF) (0.62 g, 3.9 mmol) were further added thereto, followed by stirring at 65°C for 6 hours.

The reaction was stopped with the use of an alkali, and the organic layer was distilled off under reduced pressure, followed by purification by silica gel column chromatography and recrystallization to give 4-[difluoro-(3,4,5-trifluorophenoxy)-methyl]-3,5-difluoro-4'-propylbiphenyl (1.50 g, 3.5 mmol) as a white solid. The isolation yield was 90%, and purity was 99.88%. $^1$H-NMR ($CDCl_3$) $\delta$ 7.47 (d, 2H, Ar), 7.28 (d, 2H, Ar), 7.20 (d, 2H, Ar), 6.99 (m, 2H, Ar), 2.64 (t, 2H, benzyl), 1.68 (m, 2H, $CH_2$), 0.97 (m, 3H, $CH_3$). $^{19}$F-NMR ($CDCl_3$) $\delta$ -58.85 (t, 2F, $CF_2O$), -105.89 (m, 2F, Ar), -127.77 (m, 2F, Ar), -158.50 (m, 1F, Ar).

Example 2

4-[Difluoro-(3,4,5-trifluorophenoxy)-methyl]-3,5-difluoro-4'-pentylbiphenyl was obtained as a white solid by performing the reaction and purification as in Example 1, except that the starting material was changed to 3,5-difluoro-4'-pentylbiphenyl-4-thionocarboxylic acid 3,4,5-trifluorophenyl ester. The isolation yield was 89% and purity was 99.90%. $^1$H-NMR ($CDCl_3$) $\delta$ 7.50 (d, 2H, Ar), 7.28 (d, 2H, Ar), 7.21 (d, 2H, Ar), 6.94 (m, 2H, Ar), 2.60 (t, 2H, benzyl), 1.71 (m, 2H, $CH_2$), 1.40 (m, 2H, $CH_2$), 1.30 (m, 2H, $CH_2$), 0.90 (t, 3H, $CH_3$). $^{19}$F-NMR ($CDCl_3$) $\delta$ -58.88(t, 2F, $CF_2O$), -105.90 (m, 2F, Ar), -127.77 (m, 2F, Ar), -158.49 (m, 1F, Ar).

Example 3

(i) Production Step of Arylthionocarboxylic Acid Aryl Ester

A 200-mL, three-necked flask was purged with nitrogen, and 1-bromo-3,5-difluorobenzene (10.0 g, 51.8 mmol) and tetrahydrofuran (THF) (9 mL) were added thereto. After being cooled to -78°C, lithium diisopropylamide (a THF/heptane/ethylbenzene solution, 34.5 mL, 51.8 mmol) was added thereto, and CuCl (5.13 g, 51.8 mmol) was further added, followed by stirring for one hour. Subsequently, 3,4,5-trifluorophenyl-chlorothioformate (11.7 g, 51.8 mmol) was added and stirred for one hour to complete the reaction.

The reaction liquid was extracted with toluene/hydrochloric acid water, and the organic layer was distilled off under reduced pressure. The obtained solid was purified by recrystallization to give 4-bromo-2,6-difluoro-thionobenzoic acid 3,4,5-trifluorophenyl ester (16.5 g, 43.0 mmol) as a yellow solid. The isolation yield was 83%. $^1$H-NMR ($CDCl_3$) $\delta$ 7.22(m, 2H, Ar), 6.85 (m, 2H, Ar). $^{19}$F-NMR ($CDCl_3$) $\delta$-111.52 (d, 2F, Ar), -132.73 (m, 2F, Ar), -162.50 (m, 1F, Ar).

(ii) Fluorination Step of Arylthionocarboxylic Acid Aryl Ester

4-Bromo-2,6-difluorothiono benzoic acid 3,4,5-trifluorophenyl ester (10 g, 26.1 mmol) obtained in step (i) above and acetonitrile (15 mL) were placed in a fluororesin container, to which iodine pentafluoride ($IF_5$) (5.79 g, 26.1 mmol) and triethylamine-trihydrofluoride salt ($NEt_3$-3HF) (4.20 g, 26.1 mmol) were further added, followed by stirring at 65°C for 4 hours.

Thereafter, purification was performed as in Example 1 to give 4-[difluoro-(3,4,5-trifluorophenoxy)-methyl]-1-bromo-3,5-difluorobenzene (8.92 g, 22.9 mmol) as a white solid. The isolation yield was 88% and purity was 99.82%. $^1$H-NMR ($CDCl_3$) $\delta$ 7.19 (m, 2H, Ar), 6.94 (m, 2H, Ar). $^{19}$F-NMR ($CDCl_3$) $\delta$ -62.98 (t, 2F, $CF_2O$), -109.82 (m, 2F, Ar), -133.23 (m, 2F, Ar), -163.75 (m, 1F, Ar).

(iii) Coupling Reaction Step

4-[Difluoro-(3,4,5-trifluoro-phenoxy)-methyl]-1-bromo-3,5-difluorobenzene (10.0 g, 25.7 mmol) obtained in step (ii) above, 4-propylphenylboronic acid (4.63 g, 28.3 mmol), $K_2CO_3$ (8.51 g, 61.7 mmol), tetrakis(triphenylphosphine)palladium (148 mg, 0.129 mmol), and 2-dicyclohexylphosphino-2',6'-dimethoxybiphenyl (53.6 mg, 0.129 mmol) were sequentially placed in a 200-mL three-necked flask, and the system was purged with nitrogen. Thereafter, degassed DMAc (10 mL) and degassed water (30 mL) were added thereto, followed by stirring at a reaction-liquid temperature of 85°C for 5 hours. The obtained crude product was extracted with toluene, the organic layer was washed with an aqueous $NaHCO_3$ solution, and then with water, followed by concentration. The obtained crude product was purified by silica chromatography and recrystallization to give 4-[difluoro-(3,4,5-trifluorophenoxy-methyl]-3,5-difluoro-4'-propylbiphenyl (9.03 g, 21.1 mmol) as a white solid. The following spectrum measurement results strongly support the structure of this compound.
$^1$H-NMR ($CDCl_3$) $\delta$ 7.47 (d, 2H, Ar), 7.28 (d, 2H, Ar), 7.20 (d, 2H, Ar), 6.99 (m, 2H, Ar), 2.64 (t, 2H, benzyl), 1.68 (m, 2H, $CH_2$), 0.97 (m, 3H, $CH_3$). $^{19}$F-NMR ($CDCl_3$) $\delta$ -58.85 (t, 2F, $CF_2O$), -105.89 (m, 2F, Ar), -127.77 (m, 2F, Ar), -158.50 (m, 1F, Ar).

Example 3-2

4-[Difluoro-(3,4,5-trifluoro-phenoxy)-methyl]-3,5-difluorophenylboronic acid (5.0 g, 14.1 mmol), 1-bromo-4-propylbenzene (2.55 g, 12.8 mmol), $K_2CO_3$ (4.25 g, 30.7 mmol), tetrabutylammonium bromide (TBAB) (412 mg, 1.28 mmol), and bis(di-tert-butyl(4-dimethylamino)phosphine)dichloropalladium (II) (9.1 mg, 0.0128 mmol) were sequentially placed in a reactor, and the system was purged with nitrogen. Thereafter, degassed DMAc (3.8 mL) and degassed water (11.3 mL) were added thereto, followed by stirring at a reaction-liquid temperature of 85°C for 4 hours. The obtained crude product was purified to give 4-[difluoro-(3,4,5-trifluorophenoxy-methyl]-3,5-difluoro-4'-propylbiphenyl (4.84 g, 11.3 mmol)

as a white solid. The isolation yield was 88.3%. The following spectrum measurement results support the structure of this compound.
$^1$H-NMR ($CDCl_3$) δ 7.47 (d, 2H, Ar), 7.28 (d, 2H, Ar), 7.20 (d, 2H, Ar), 6.99 (m, 2H, Ar), 2.64 (t, 2H, benzyl), 1.68 (m, 2H, $CH_2$), 0.97 (m, 3H, $CH_3$) . $^{19}$F-NMR ($CDCl_3$) δ -58.85 (t, 2F, $CF_2O$), -105.89 (m, 2F, Ar), -127.77 (m, 2F, Ar), -158.50 (m, 1F, Ar).

Example 4

(i) Production Process of Arylthionocarboxylic Acid Aryl Ester

Metal magnesium (0.95 g, 39.2 mmol) and THF (10 mL) were placed in a 200-mL, three-necked flask, which had been purged with nitrogen, and a small amount of iodine was added thereto and stirred. Then, p-bromochlorobenzene (5.00 g, 26.1 mmol) was added at room temperature, followed by gentle stirring. After stirring under reflux for 8 hours, the mixture was cooled to room temperature to give a solution of 4-chlorophenyl magnesium bromide (Grignard reagent) in THF. Subsequently, 3,4,5-trifluorophenyl-chlorothioformate (5.92 g, 26.1 mmol) was added thereto and stirred for one hour to complete the reaction.
Thereafter, purification was performed as in Example 3 to give 4-chlorothionobenzoic acid-3,4,5-trifluorophenyl ester (6.72 g, 22.2 mol) as a yellow solid. The isolation yield was 85%. $^1$H-NMR ($CDCl_3$) 58.07 (m, 2H, Ar), 7.51 (m, 2H, Ar), 7.17 (m, 2H, Ar). $^{19}$F NMR ($CDCl_3$) δ -132.85 (Ar, 2F), -162.58 (Ar, 1F).

(ii) Fluorination Step of Arylthionocarboxylic Acid Aryl Ester

The reaction and purification were performed as in the fluorination step of arylthionocarboxylic acid aryl ester in Example 3, except that 4-chlorothionobenzoic acid 3,4,5-trifluorophenyl ester obtained in the above step was used.
As a result, 4-[difluoro-(3,4,5-trifluorophenoxy)-methyl]-1-chlorobenzene was obtained as a white solid. The isolation yield was 92% and purity was 99.86%. $^1$H-NMR ($CDCl_3$) δ7.42 (m, 2H, Ar), 7.39 (m, 2H, Ar), 6.96 (m, 2H, Ar). $^{19}$F-NMR ($CDCl_3$) δ-62.03 (d, 2F, $CF_2O$), -134.49 (m, 2F, Ar), -163.56 (m, 1F, Ar).

(iii) Coupling Reaction Step

4-[Difluoro-(3,4,5-trifluorophenoxy)-methyl]-1-chlorobenzene (10.0 g, 32.4 mmol) obtained in step (ii) above, 4-(4-propylcyclohexyl)phenylboronic acid (8.37 g, 34.0 mmol), $K_2CO_3$ (13.43 g, 97.2 mmol), tetrabutylammonium bromide (TBAB) (522 mg, 1.62 mmol), bis(di-tert-butyl(4-dimethylaminophenyl)phosphine)dichloropalladium (II) (22.9 mg, 0.0324 mmol) were sequentially placed in a 200-mL SUS autoclave, and the system was purged with nitrogen. Thereafter, degassed DMAc (10 mL) and degassed water (30 mL) were added thereto, followed by stirring at a reaction-liquid temperature of 98°C for 5 hours. Then, purification was performed as in step (iii) of Example 3 to give 4-[difluoro-(3,4,5-trifluorophenoxy)-methyl]-4'-(4-propylcyclohexyl)biphenyl (13.2 g, 27.9 mmol) as a white solid. The isolation yield was 86%. The following spectrum measurement results strongly support the structure of this compound.
$^1$H-NMR ($CDCl_3$) δ7.54 (m, 2H, Ar), 7.46 (m, 2H, Ar), 7.42 (m, 2H, Ar), 7.39 (d, 2H, Ar), 6.88 (d, 2H, Ar), 6.99 (m, 2H, Ar), 2.69 (m, 1H, Cy), 1.79 (m, 2H, Cy), 1.84 (m, 1H, Cy), 1.31 (m, 2H, Cy), 1.27 (t, 2H, $CH_2$), 1.15 (m, 2H, $CH_2$), 0.88 (m, 3H, $CH_3$). $^{19}$F-NMR ($CDCl_3$) δ-63.00 (t, 2F, $CF_2O$), -132.70 (d, 2F, Ar), -164.40 (t, 1F, Ar) .

Example 5

(i) Production Step of Arylthionocarboxylic Acid Aryl Ester

A 200-mL, three-necked flask was purged with nitrogen, and 1-chloro-3,5-difluorobenzene (10.0 g, 67.3 mmol) and tetrahydrofuran (THF) (9 mL) were added thereto. After cooling to -78°C, an N-butyllithium heptane solution (46.0 mL, 69.0 mmol) was added, and CuCl (6.66 g, 67.3 mmol) was further added, followed by stirring for one hour. Subsequently, 3,4,5-trifluorophenyl-chlorothioformate (15.2 g, 67.3 mmol) was added thereto and stirred for one hour to complete the reaction.
Thereafter, purification was performed as in step (i) of Example 3 to give 4-chloro-2,6-difluoro-thionobenzoic acid 3,4,5-trifluorophenyl ester (20.0 g, 59.2 mmol) as a yellow solid. The isolation yield was 88%.
$^1$H-NMR ($CDCl_3$) δ7. 06 (d, 2H, Ar), 6.85 (m, 2H, Ar) . $^{19}$F-NMR ($CDCl_3$) δ-111.32 (d, 2F, Ar), -132.73 (m, 2F, Ar), -162.50 (m, 1F, Ar).

(ii) Fluorination Step of Arylthionocarboxylic Acid Aryl Ester

The reaction and purification were performed as in step (ii) of Example 3, except that 4-chloro-2,6-difluorothiono benzoic acid 3,4,5-trifluorophenyl ester obtained in step (i) above was used.

As a result, 4-[difluoro-(3,4,5-trifluorophenoxy)-methyl]-1-chloro-3,5-difluorobenzene was obtained as a white solid. The isolation yield was 90% and purity was 99.85%. $^1$H-NMR ($CDCl_3$) $\delta$7.05 (d, 2H, Ar), 6.96 (m, 2H, Ar) . $^{19}$F NMR ($CDCl_3$) : $\delta$-62.94 (t, 2F, $CF_2O$), -109.74 (m, 2F, Ar), -133.30 (m, 2F, Ar), -163.88 (m, 2F, Ar).

(iii) Coupling Reaction Step

4-[Difluoro-(3,4,5-trifluorophenoxy)-methyl]-1-chloro-3,5-difluorobenzene (10.1 g, 29.3 mmol) obtained in step (ii) above, 4-propylphenylboronic acid (5.01 g, 30.8 mmol), $K_2CO_3$ (12.1 g, 87.9 mmol), tetrabutylammonium bromide (TBAB) (472 mg, 1.47 mmol), bis(di-tert-butyl(4-dimethylaminophenyl)phosphine)dichloropalladium(II) (20.7 mg, 0.0293 mmol) were sequentially placed in a 200-mL three-necked flask, and the system was purged with nitrogen. Thereafter, degassed water (40 mL) was added thereto, followed by stirring at a reaction-liquid temperature of 90°C for 3 hours. Then, purification was performed as in step (iii) of Example 3 to give a white solid (11.0 g, 25.6 mmol). The following spectrum measurement results are completely the same as those of Example 3, and thus strongly support the structure of this compound. $^1$H-NMR ($CDCl_3$) $\delta$ 7.47 (d, 2H, Ar), 7.28 (d, 2H, Ar), 7.20 (d, 2H, Ar), 6.99 (m, 2H, Ar), 2.64 (t, 2H, benzyl), 1.68 (m, 2H, $CH_2$), 0.97 (m, 3H, $CH_3$) . $^{19}$F-NMR ($CDCl_3$) $\delta$ -58.85 (t, 2F, $CF_2O$), -105.89 (m, 2F, Ar), -127.77 (m, 2F, Ar), -158.50 (m, 1F, Ar).

Comparative Example 1

(4-Methoxyphenyl)thioacetic acid O-ethyl (2.1 g, 10 mmol) used as a starting material was placed into a fluororesin container, ethyl acetate (8 mL) was added thereto, and iodine pentafluoride ($IF_5$) (2.66 g, 12 mmol) and triethylamine-trihydrofluoride salt ($NEt_3$-3HF) (1.93 g, 12 mmol) were further added, followed by stirring at room temperature for 2.5 hours. Thereafter, purification was performed as in Example 1.

As a result, a white solid comprising 1-(2-ethoxy-2,2-difluoroethyl)-4-methoxybenzene (1.8 g) was obtained. However, the reaction product contained an impurity, i.e., 1-(2-ethoxy-2,2-difluoro-1-iodoethyl)-4-methoxybenzene in which iodine was added at the benzylic position of the starting compound, and the GC purity was 95.20%. This impurity was difficult to remove by a general purification method, and the product was thus not suitable for use as a compound such as for liquid crystal.

In contrast, as is clear from the results of the fluorination steps of Examples 1 to 5, when arylthionocarboxylic acid aryl ester used as a starting material was subjected to fluorination with iodine pentafluoride, and a general purification method, such as silica gel column chromatography and recrystallization, was performed, almost all of impurities were removed, and a reaction product was easily obtained at a very high purity.

## Claims

1. A method for producing a compound of the formula

$$Ar^2\text{-}CF_2\text{-}O\text{-}Ar^1 \qquad (2)$$

wherein

$Ar^1$ is an aromatic ring group, cycloalkyl or alkyl, each optionally substituted,
$Ar^2$ is

(a) an optionally substituted aromatic ring group,
(b) an optionally substituted cycloalkyl, or
(c) an alkyl optionally substituted by one or more groups selected from cycloalkyl, fluoroalkyl, alkoxy, fluoroalkoxy, halogen, $-SF_5$, and a boronic acid group, comprising reacting a compound of the formula

$$Ar^2\text{-}C(=S)\text{-}O\text{-}Ar^1 \qquad (1)$$

wherein $Ar^1$ and $Ar^2$ are as defined above, with a compound of the formula $IF_n$, wherein n is a natural number of 1-5.

**2.** The method of claim 1, which further comprises a step of preparing a compound of the formula $Ar^2$-C(=S)-O-$Ar^1$ (1), wherein $Ar^1$ and $Ar^2$ are as defined in claim 1, by reacting a compound of the formula X-C(=S)-O-$Ar^1$ (3) wherein $Ar^1$ is as defined above, and X is a halogen atom, with a compound of the formula $Ar^2$-M (4), wherein $Ar^2$ is as defined above, and M is an alkali metal, monovalent copper or -Mg$Y^1$, wherein $Y^1$ is halogen.

**3.** A method for producing a compound of formula (9)

$$Ar^5\text{-}Ar^6\text{-}Z\text{-}Ar^7 \qquad (9)$$

wherein $Ar^5$ and $Ar^7$ each independently is an aromatic ring group, cycloalkyl or alkyl, each optionally substituted, $Ar^6$ is an optionally substituted divalent hydrocarbon group, and
Z is -$CF_2$-O- or -O-$CF_2$-,

comprising the steps of

(i) reacting a compound of the formula $Ar^3$-C(=S)-O-$Ar^4$ (1-1) wherein

- one of $Ar^3$ and $Ar^4$ is a group substituted with a group or atom having reactivity with a boronic acid compound, or represents a group substituted with a boronic acid group, and
- the other is an aromatic ring group, cycloalkyl or alkyl, each optionally substituted,

with a compound of the formula $IF_n$, wherein n is a natural number of 1-5, to obtain a compound of the formula $Ar^3$-$CF_2$-O-$Ar^4$ (2-1) wherein $Ar^3$ and $Ar^4$ are as defined above; and
(ii) reacting the obtained compound of the formula (2-1) with a compound of the formula $Ar^5$-D (8) wherein D is

- a boronic acid group when the compound of the formula (2-1) is a compound containing a group or atom having reactivity with a boronic acid compound,
- a group having reactivity with a boronic acid compound when the compound of the formula (2-1) is a compound containing a boronic acid group,

and $Ar^5$ is an aromatic ring group, cycloalkyl or alkyl, each optionally substituted, to obtain a compound of the formula (9).

**4.** The method of claim 3, which further comprises a step of preparing a compound of the formula $Ar^3$-C(=S)-O-$Ar^4$ (1-1) wherein $Ar^3$ and $Ar^4$ are as defined in claim 3, by reacting a compound of the formula X-C(=S)-O-$Ar^4$ (3-1) wherein $Ar^4$ is as defined above, and X is a halogen atom, with a compound of the formula $Ar^3$-M (4), wherein $Ar^3$ is as defined above, and M is an alkali metal, monovalent copper or -Mg$Y^1$, wherein $Y^1$ is halogen.

**5.** The method of claim 3 or 4, wherein the boronic acid group is a group of the formula

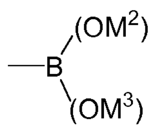

(a)

wherein $M^2$ and $M^3$ each independently are H, an alkyl group having six or less carbon atoms, or a monovalent metal atom, or $M^2$ and $M^3$ may bond to each other to form a divalent aliphatic hydrocarbon group, and taken together with the boron atom, may form a ring structure.

**Patentansprüche**

**1.** Verfahren zur Herstellung einer Verbindung der Formel

$$Ar^2\text{-}CF_2\text{-}O\text{-}Ar^1 \qquad (2)$$

worin

$Ar^1$ eine aromatische Ringgruppe, Cycloalkyl oder Alkyl ist, jeweils optional substituiert,
$Ar^2$

(a) eine optional substituierte aromatische Ringgruppe,
(b) ein optional substituiertes Cycloalkyl oder
(c) ein Alkyl, optional substituiert mit einer oder mehr Gruppen, ausgewählt aus Cycloalkyl, Fluoralkyl, Alkoxy, Fluoralkoxy, Halogen, $-SF_5$ und einer Boronsäuregruppe,

ist, umfassend Umsetzen einer Verbindung der Formel

$Ar^2$-C(=S)-O-$Ar^1$ (1)

worin $Ar^1$ und $Ar^2$ wie vorstehend definiert sind, mit einer Verbindung der Formel $IF_n$, worin n eine natürliche Zahl von 1 bis 5 ist.

**2.** Verfahren gemäß Anspruch 1, welches ferner einen Schritt des Zubereitens einer Verbindung der Formel

$Ar^2$-C(=S)-O-$Ar^1$ (1)

umfasst, worin $Ar^1$ und $Ar^2$ wie in Anspruch 1 definiert sind, indem eine Verbindung der Formel

X-C(=S)-O-$Ar^1$ (3),

worin $Ar^1$ wie vorstehend definiert ist und X ein Halogenatom ist, mit einer Verbindung der Formel $Ar^2$-M (4) umgesetzt wird, worin $Ar^2$ wie vorstehend definiert ist, und M ein Alkalimetall, monovalentes Kupfer oder -$MgY^1$ ist, worin $Y^1$ Halogen ist.

**3.** Verfahren zur Herstellung einer Verbindung der Formel (9)

$Ar^5$-$Ar^6$-Z-$Ar^7$ (9)

worin $Ar^5$ und $Ar^7$ jeweils unabhängig eine aromatische Ringgruppe, Cycloalkyl oder Alkyl sind, jeweils optional substituiert, $Ar^6$ eine optional substituierte divalente Kohlenwasserstoffgruppe ist und Z $-CF_2-O-$ oder $-O-CF_2-$ ist, umfassend die Schritte

(i) Umsetzen einer Verbindung der Formel

$Ar^3$-C(=S)-O-$Ar^4$ (1-1)

worin

- eines von $Ar^3$ und $Ar^4$ eine Gruppe ist, die mit einer Gruppe oder einem Atom substituiert ist, die/das Reaktivität mit einer Boronsäureverbindung aufweist, oder eine Gruppe darstellt, die mit einer Boronsäuregruppe substituiert ist, und
- das andere eine aromatische Ringgruppe, Cycloalkyl oder Alkyl ist, jeweils optional substituiert,

mit einer Verbindung der Formel $IF_n$, worin n eine natürliche Zahl von 1 bis 5 ist, um eine Verbindung der Formel $Ar^3$-$CF_2$-O-$Ar^4$ (2-1) zu erhalten, worin $Ar^3$ und $Ar^4$ wie vorstehend definiert sind; und
(ii) Umsetzen der erhaltenen Verbindung (2-1) mit einer Verbindung der Formel $Ar^5$-D (8), worin D

- eine Boronsäuregruppe ist, wenn die Verbindung der Formel (2-1) eine Verbindung ist, die eine Gruppe oder ein Atom enthält, die/das Reaktivität mit einer Boronsäurebindung aufweist,
- eine Gruppe ist, die Reaktivität mit einer Boronsäureverbindung aufweist, wenn die Verbindung der Formel (2-1) eine Verbindung ist, die eine Boronsäuregruppe enthält,

und $Ar^5$ eine aromatische Ringgruppe, Cycloalkyl oder Alkyl ist, jeweils optional substituiert, um eine Verbindung der Formel (9) zu erhalten.

**4.** Verfahren gemäß Anspruch 3, welches Ferner einen Schritt zur Zubereitung einer Verbindung der Formel $Ar^3$-C(=S)-O-$Ar^4$ (1-1) umfasst, worin $Ar^3$ und $Ar^4$ wie in Anspruch 3 definiert sind, indem eine Verbindung der Formel X-C(=S)-O-$Ar^4$ (3-1), worin $Ar^4$ wie vorstehend definiert ist und X ein Halogenatom ist, mit einer Verbindung der Formel $Ar^3$-M (4) umgesetzt wird, worin $Ar^3$ wie vorstehend definiert ist und M ein Alkalimetall, monovalentes Kupfer oder -$MgY^1$ ist, worin $Y^1$ ein Halogen ist.

**5.** Verfahren gemäß Anspruch 3 oder 4, worin die Boronsäuregruppe eine Gruppe der Formel

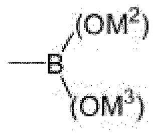 (a)

ist, worin $M^2$ und $M^3$ jeweils unabhängig H, eine Alkylgruppe mit sechs oder weniger Kohlenstoffatomen oder ein monovalentes Metallatom sind, oder $M^2$ oder $M^3$ miteinander binden können, um eine divalente aliphatische Kohlenwasserstoffgruppe zu bilden, und zusammen mit dem Boratom eine Ringstruktur bilden können.

## Revendications

**1.** Procédé de production d'un composé de la formule

$Ar^2$-CF2O-$Ar^1$ (2)

dans laquelle

$Ar^1$ représente un groupement cyclique aromatique, un cycloalkyle ou un alkyle, chacun étant éventuellement substitué,
$Ar^2$ représente

(a) un groupement cyclique aromatique éventuellement substitué,
(b) un cycloalkyle éventuellement substitué, ou
(c) un alkyle éventuellement substitué par un ou plusieurs groupements choisis parmi un groupement cycloalkyle, un groupement fluoroalkyle, un groupement alcoxy, un groupement fluoroalcoxy, un groupement halogène, un groupement -$SF_5$ et un groupement acide boronique,

comprenant la réaction d'un composé de la formule

$Ar^2$-C(=S)-O-Ar1 (1)

dans laquelle $Ar^1$ et $Ar^2$ sont tels que définis ci-dessus, avec un composé de la formule $IF_n$, dans laquelle $n$ représente un nombre naturel de 1 à 5.

**2.** Procédé selon la revendication 1, qui comprend en outre une étape de préparation d'un composé de la formule $Ar^2$-C(=S)-O-$Ar^1$ (1),
dans laquelle $Ar^1$ et $Ar^2$ sont tels que définis dans la revendication 1, en faisant réagir un composé de la formule X-C(=S)-O-$Ar^1$ (3) dans laquelle $Ar^1$ est tel que défini ci-dessus, et X représente un atome d'halogène, avec un composé de la formule $Ar^2$-M (4) dans laquelle $Ar^2$ est tel que défini ci-dessus, et M est un métal alcalin, du cuivre monovalent ou -$MgY^1$, où $Y^1$ représente de l'halogène.

**3.** Procédé de production d'un composé de formule (9)

$Ar^5$-$Ar^6$-Z-$Ar^7$ (9)

dans laquelle $Ar^5$ et $Ar^7$ représentent chacun indépendamment un groupement cyclique aromatique, un cycloalkyle ou un alkyle, chacun étant éventuellement substitué, $Ar^6$ représente un groupement hydrocarboné divalent éventuellement substitué, et Z représente -$CF_2$-O- ou -O-$CF_2$-,

comprenant les étapes de

(i) mise en réaction d'un composé de la formule $Ar^3$-C(=S)-O-$Ar^4$ (1-1) dans laquelle

- un de $Ar^3$ et $Ar^4$ représente un groupement substitué par un groupement ou un atome ayant une réactivité avec un composé d'acide boronique, ou représente un groupement substitué par un groupement acide boronique, et
- l'autre représente un groupement cyclique aromatique, un cycloalkyle ou un alkyle, chacun étant éventuellement substitué,

avec un composé de la formule $IF_n$, dans laquelle *n* représente un nombre naturel de 1 à 5, pour obtenir un composé de la formule $Ar^3$-$CF_2$O-$Ar^4$ (2-1) dans laquelle $Ar^3$ et $Ar^4$ sont tels que définis ci-dessus ; et
(ii) mise en réaction du composé obtenu de la formule (2-1) avec un composé de la formule $Ar^5$-D (8) dans laquelle D représente

- un groupement acide boronique lorsque le composé de la formule (2-1) est un composé contenant un groupement ou un atome ayant une réactivité avec un composé d'acide boronique,
- un groupement ayant une réactivité avec un composé d'acide boronique lorsque le composé de la formule (2-1) est un composé contenant un groupement acide boronique,

et $Ar^5$ représente un groupement cyclique aromatique, un cycloalkyle ou un alkyle, chacun étant éventuellement substitué, pour obtenir un composé de la formule (9).

**4.** Procédé selon la revendication 3, qui comprend en outre une étape de préparation d'un composé de la formule $Ar^3$-C(=S)-O-$Ar^4$ (1-1) dans laquelle $Ar^3$ et $Ar^4$ sont tels que définis dans la revendication 3, en faisant réagir un composé de la formule X-C(=S)-O-$Ar^4$ (3-1) dans laquelle $Ar^4$ est tel que défini ci-dessus, et X représente un atome d'halogène, avec un composé de la formule $Ar^3$-M (4) dans laquelle $Ar^3$ est tel que défini ci-dessus, et M est un métal alcalin, du cuivre monovalent ou -$MgY^1$, où $Y^1$ représente de l'halogène.

**5.** Procédé selon la revendication 3 ou 4, dans lequel le groupement acide boronique est un groupement de la formule

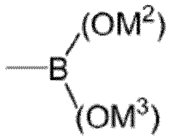

(a)

dans laquelle $M^2$ et $M^3$ représentent chacun indépendamment H, un groupement alkyle ayant six atomes de carbone ou moins, ou un atome de métal monovalent, ou $M^2$ et $M^3$ peuvent être liés l'un à l'autre pour former un groupement hydrocarboné aliphatique divalent, et pris conjointement avec l'atome de bore, peuvent former une structure cyclique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003525286 A
- JP 2003261478 A
- JP 2013241401 A
- US 20110233466 A1


### Non-patent literature cited in the description


- *J.Chem.Soc., Chem. Commun.,* 1993
- *Synthetic Communications,* 1999, vol. 29 (2), 201-210
- **H. VIOLA et al.** *Chem. Ber.,* 1968, vol. 101, 3517
- **LIEBIGS.** *Ann. Chem.,* 1973, 1637
- **A. OHNO et al.** *Tetrahedron Lett.,* 1968, 2083
- *Synthesis,* 1973, 149
- *Bull. Chem. Soc. Belg.,* 1987, vol. 87, 293
- **S. SCHEITHAUER et al.** *Chem. Ber.,* 1965, vol. 98, 838
- **LIEBIGS.** *Ann. Chem.,* 1974, 671
- *Z. Chem.,* 1966, vol. 6, 108
- **K. A. LATIF et al.** *Tetrahedron,* 1970, vol. 26, 4247